# EUROPEAN PATENT SPECIFICATION

(11) **EP 1 421 199 B1**
(45) Date of publication and mention of the grant of the patent: **28.07.2010**
(21) Application number: 02760784.5
(22) Date of filing: 29.08.2002
(51) Int. Cl.: C12N 15/864, A61K 48/00, C12N 15/12, A61P 25/00, C07K 14/475

(54) **COMPOSITIONS FOR TREATING NEURODEGENERATIVE DISEASES**
ZUSAMMENSTELLUNGEN ZUR BEHANDLUNG VON NEURODEGENERATIVEN ERKRANKUNGEN
COMPOSITIONS DESTINES AU TRAITEMENT DES MALADIES NEURODEGENERATIVES

(30) Priority: 29.08.2001 US 315838 P
(43) Date of publication of application: 26.05.2004
(73) Proprietor: Mitsubishi Tanabe Pharma Corporation, Osaka 541-8505 (JP)
(72) Inventor: OZAWA, Keiya, c/o Jichi Medical School, Kawachi-gun, Tochigi 329-0498 (JP); MURAMATSU, Shin-ichi, c/oJichi Medical School, Kawachi-gun, Tochigi 329-0498 (JP); IKEGUCHI, Kunihiko, c/o Jichi MedicalSchool, Kawachi-gun, Tochigi 329-0498 (JP); NAKAMO, Imaharu, c/o Jichi Medical School, Kawachi-gun, Tochigi 329-0498 (JP)
(74) Representative: HOFFMANN EITLE
(86) International application number: PCT/JP2002/008761
(87) International publication number: WO 2003/018821

(56) References cited:
- MANDEL ET AL: "Recombinant adeno-associated viral vector-mediated glial cell line-derived neurotrophic factor gene transfer protects nigral dopamine neurons after onset of progressive degeneration in a rat model of Parkinson's disease" EXPERIMENTAL NEUROLOGY, vol. 160, 1999, pages 205-214, XP001148187
- DURING M J ET AL: "Neuroprotection and neuroregeneration in rodent and primate Parkinsonian models using AAV vectors expressing GDNF." SOCIETY FOR NEUROSCIENCE ABSTRACTS, vol. 21, no. 1-3, 1995, page 542 XP001109631 25th Annual Meeting of the Society for Neuroscience;San Diego, California, USA; November 11-16, 1995 ISSN: 0190-5295
- DATABASE BIOSIS [Online] BIOSCIENCES INFORMATION SERVICE, PHILADELPHIA, PA, US; 2000 CHEN J F ET AL: "Novel neuroprotection by A2A adenosine receptor inactivation in the MPTP model of Parkinson's disease." Database accession no. PREV200100089843 XP002230361 & SOCIETY FOR NEUROSCIENCE ABSTRACTS, vol. 26, no. 1-2, 2000, pages Abstract No.-478.1, 30th Annual Meeting of the Society of Neuroscience;New Orleans, LA, USA; November 04-09, 2000 ISSN: 0190-5295
- MANDEL R J ET AL: "Functional recovery after intrastriatal 6-OHDA lesions mediated by intrastriatal injections of recombinant AAV encoding rat GDNF." SOCIETY FOR NEUROSCIENCE ABSTRACTS, vol. 25, no. 1-2, 1999, page 328 XP001109629 29th Annual Meeting of the Society for Neuroscience, Part 1;Miami Beach, Florida, USA; October 23-28, 1999 ISSN: 0190-5295
- MANDEL R J ET AL: "Recombinant AAV encoding rat GDNF spares nigral neurons in a progressive 6-OHDA degeneration model in rats." SOCIETY FOR NEUROSCIENCE ABSTRACTS, vol. 23, no. 1-2, 1997, page 296 XP001109630 27th Annual Meeting of the Society for Neuroscience, Part 1;New Orleans, Louisiana, USA; October 25-30, 1997 ISSN: 0190-5295
- DATABASE BIOSIS [Online] BIOSCIENCES INFORMATION SERVICE, PHILADELPHIA, PA, US; 1993 SAINT-CYR J A ET AL: "Neuropsychological and psychiatric side effects in the treatment of Parkinson's disease." Database accession no. PREV199497098230 XP002230362 & NEUROLOGY, vol. 43, no. 12 SUPPL. 6, 1993, pages S47-S52, ISSN: 0028-3878
- DATABASE BIOSIS [Online] BIOSCIENCES INFORMATION SERVICE, PHILADELPHIA, PA, US; March 2002 (2002-03) WANG L ET AL: "Delayed delivery of AAV-GDNF prevents nigral neurodegeneration and promotes functional recovery in a rat model of Parkinson's disease." Database accession no. PREV200200261759 XP002230363 & GENE THERAPY, vol. 9, no. 6, March 2002 (2002-03), pages 381-389, March, 2002 ISSN: 0969-7128
- DATABASE BIOSIS [Online] BIOSCIENCES INFORMATION SERVICE, PHILADELPHIA, PA, US; 2001 QUINTERO E M ET AL: "Adeno-associated virus transfected with GDNF alters behavior and restores function to dopaminergic neurons in 6-OHDA lesioned rats." Database accession no. PREV200100497468 XP002230364 & SOCIETY FOR NEUROSCIENCE ABSTRACTS, vol. 27, no. 1, 2001, page 525 31st Annual Meeting of the Society for Neuroscience;San Diego, California, USA; November 10-15, 2001 ISSN: 0190-5295

## Description

### Technical Field

The present invention relates generally to compositions for gene delivery. In particular, the present invention pertains to adeno-associated virus (AAV)-based gene delivery systems for delivering glial cell line-derived neutrotrophic factor (GDNF) to treat preexisting neuronal damage caused by neurodegenerative conditions such as Parkinson's disease.

### Background of the Invention

Central nervous system (CNS) disorders present major public health issues. Parkinson's disease (PD) alone affects over one million people in the United States. Clinically, PD is characterized by a decrease in spontaneous movements, gait difficulty, postural instability, rigidity and tremor. PD is a progressive neurodegenerative disorder that predominantly affects dopaminergic (DA) neurons in the substantia nigra (SN). Currently, many CNS disorders are treated by systemic administration of a therapeutic agent. Systemic administration, however, is often inefficient because of the inability of drugs to pass through the blood-brain barrier. Even if these compounds are successful in penetrating the blood-brain-barrier, they may induce central nervous system side effects. Thus, many potentially useful compounds, such as proteins, cannot be administered systemically.

Currently available therapies of PD aim at replacing dopamine in the striatum to restore motor functions. However, it is essential to develop therapeutic interventions that block or slowdown the ongoing degenerative process (Dunnett et al., Nature (1999) 399:A32-39). Glial cell line-derived neurotrophic factor (GDNF) is the most potent neurotrophic factor discovered to date for DA neurons, and has been shown to enhance DA neuron survival both *in vitro* and *in vivo* (Bjorklund et al., Brain Res. (2000) 886:82-98; Bohn, M.C., Mol. Ther. (2000) 1:494-496; Ozawa et al., J. Neural Transm. Suppl. (2000) 58:181-191). However, administration of GDNF protein to the central nervous system is problematic. GDNF remains active for only a short period of time and due to its relatively large size, GDNF does not cross the blood-brain barrier, making direct administration into the brain the only viable means of delivery. This necessitates stereotaxic injection of GDNF into the brain or intracerebroventricular (ICV) delivery which severely limits its therapeutic utility. Direct *in vivo* gene transfer, therefore, offers a more efficient method of GDNF delivery.

Adeno-associated virus (AAV) has been used with success to deliver genes for gene therapy. The AAV genome is a linear, single-stranded DNA molecule containing about 4681 nucleotides. The AAV genome generally comprises an internal nonrepeating genome flanked on each end by inverted terminal repeats (ITRs). The ITRs are approximately 145 base pairs (bp) in length. The ITRs have multiple functions, including as origins of DNA replication, and as packaging signals for the viral genome. The internal nonrepeated portion of the genome includes two large open reading frames, known as the AAV replication (*rep*) and capsid (*cap*) genes. The *rep* and *cap* genes code for viral proteins that allow the virus to replicate and package into a virion. In particular, a family of at least four viral proteins are expressed from the AAV *rep* region, Rep 78, Rep 68, Rep 52, and Rep 40, named according to their apparent molecular weight. The AAV *cap* region encodes at least three proteins, VPI, VP2, and VP3.

AAV has been engineered to deliver genes of interest by deleting the internal nonrepeating portion of the AAV genome (i.e., the *rep* and *cap* genes) and inserting a heterologous gene between the ITRs. The heterologous gene is typically functionally linked to a heterologous promoter (constitutive, cell-specific, or inducible) capable of driving gene expression in the patient's target cells under appropriate conditions. Termination signals, such as polyadenylation sites, can also be included.

AAV is a helper-dependent virus; that is, it requires coinfection with a helper virus (e.g.; adenovirus, herpesvirus or vaccinia), in order to form AAV virions. In the absence of coinfection with a helper virus, AAV establishes a latent state in which the viral genome inserts into a host cell chromosome, but infectious virions are not produced. Subsequent infection by a helper virus "rescues" the integrated genome, allowing it to replicate and package its genome into an infectious AAV virion. While AAV can infect cells from different species, the helper virus must be of the same species as the host cell. Thus, for example, human AAV will replicate in canine cells coinfected with a canine adenovirus.

Studies have demonstrated that delivery of the GDNF gene using AAV, adenoviral (Ad) or lentiviral vector-based systems protects nigral DA neurons while regenerating the nigrostriatal pathway in rodent and primate models of PD if administered before or shortly after an injection of neurotoxin (Choi-Lundberg et al., Science (1997) 275:838-841; Mandel et al., Proc. Natl. Acad. Sci. USA (1997) 94:14083-14088; Bilang-Bieuel et al., Proc. Natl. Acad. Sci. USA (1997) 94:8818-8823; Choi-Lundberg et al., Exp. Neurol. (1998) 154:261-275; Mandel et al., Exp. Neurol. (1999) 160:205-214; Kirik et al., J. Neurosci (2000) 20:4686-4700; Bensadoun et al., Exp. Neurol. (2000) 164:15-24; and Kordower et al., Science (2000) 290:767-773). However, previous studies have shown that when GDNF is administered with a delay after the insult, sparing of DA neurons is only marginal and the magnitude of functional recovery probably reflects the number of DA neurons still surviving and maintaining nigrostriatal connections (Kirik et al., J. Neurosci. (2000) 20:4686-4700; Connor et al., Gene Therapy (1999) 6:1936-1951; Winkler et al., J. Neurosci. (1996) 16:7206-7215; Natsume et al., Exp. Neurol. (2001) 169:231-238). Thus, the effectiveness of chronically delivered GDNF gene via AAV vector-based systems in a later phase of the degenerative process has not previously been documented.

PD is a disorder characterized by progressive DA degeneration, and substantial numbers of DA neurons are depleted before the obvious appearance of symptoms. It is thus clinically more important to address whether GDNF gene delivered in a delayed manner is capable of rescuing the DA neurons and improving behavioral performance in animal models with extensive nigrostriatal DA denervation already present. In addition, it is not clear whether vector-derived GDNF could be retrogradely transported from axon terminals to cell bodies of DA neurons in the SN.

### Summary of the Invention

There is a need in the art for an alternative means to deliver GDNF in order to treat preexisting neuronal damage. The present invention is based on the discovery that AAV vector-mediated GDNF gene delivery to subjects with extensive nigrostriatal dopaminergic (DA) denervation, promotes the survival of DA neurons in the substantia nigra (SA), increases the density of tyrosine hydroxylase (TH)-positive DA fibers, and ameliorates behavioral and biochemical deficits even after the degenerative process has begun. Moreover, transgene-derived GDNF is retrogradely transported from the striatum to DA cell bodies in the SN, while maintaining functional connections within the nigrostriatal pathway, even after substantial numbers of DA cells have been lost.

Accordingly, the present invention provides the following:
1. Use of recombinant adeno-associated virus (AAV) virions, wherein said virions comprise a polynucleotide encoding a glial cell line-derived neutrotrophic factor (GDNF) polypeptide operably linked to expression control elements that comprise a promoter, for the preparation of a medicament for the treatment in a mammalian subject of a central nervous system disorder characterized by the existence in said disorder of pre-existing neuronal damage comprising moderate to extensive nigrostriatal dopaminergic (DA) denervation;
2. The use of item 1, wherein said central nervous system disorder is Parkinson's disease (PD);
3. The use of any items 1 and 2 wherein the medicament is in a form for administration to the striatum of said subject.

The recombinant AAV virions can be administered to the striatum using convection-enhanced delivery (CED).

In certain embodiments, the subject is a human.

The recombinant AAV virions may be administered to the striatum of the subject using CED.

The promoter may be a viral promoter, such as an MLP, CMV, or RSV LTR promoter.

If CED is used, the administering may be done with an osmotic pump or an infusion pump.

The polynucleotide in any of the methods may further comprise a secretory sequence in the 5' position and in reading frame with the sequence encoding the GDNF polypeptide. Additionally, the polynucleotide may encode a human GDNF, such as a human pre-pro-GDNF.

The present invention also provides a composition for treating a mammalian subject with a disease or disorder as described above, comprising recombinant AAV virions that comprise a polynucleotide encoding a GDNF polypeptide operably linked to expression control elements that comprise a promoter, under conditions that result in transduction of neural cells in vivo to provide a therapeutic effect. The composition of the present invention can be a pharmaceutical composition optionally comprising a pharmaceutically acceptable carrier.

Accordingly, the present invention provides the following:
1. Use of recombinant adeno-associated virus (AAV) virions, wherein said virions comprise a polynucleotide encoding a glial cell line-derived neutrotrophic factor (GDNF) polypeptide operably linked to expression control elements that comprise a promoter, for the preparation of a medicament for the treatment in a mammalian subject of a central nervous system disorder characterized by the existence in said disorder of pre-existing neuronal damage comprising moderate to extensive nigrostriatal dopaminergic (DA) denervation.
2. The use of item 1, wherein said central nervous system disorder is Parkinson's disease (PD).
3. The use of any items 1 and 2, wherein the medicament is in a form for administration to the striatum of said subject.

These and other embodiments of the subject invention will readily occur to those of skill in the art in view of the disclosure herein.

### Brief Description of the Figures

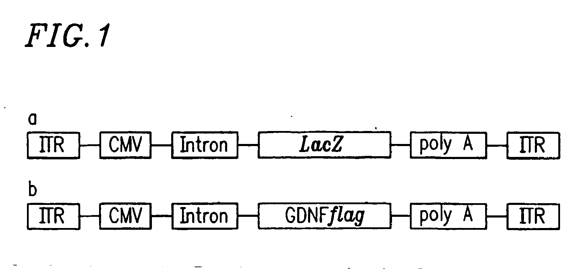

Figures 1a and 1b are schematic illustrations of AAV vectors. In Figure 1a, *LacZ* is under the transcriptional control of CMV promoter. In Figure 1b, mouse GDNF cDNA is fused with the coding sequence of the FLAG peptide at the C-terminus and is under the transcriptional control of the CMV promoter. ITR, inverted terminal repeat; CMV, cytomegalovirus immediate-early promoter; intron, first intron of human growth hormone; poly A, SV40 polyadenylation signal sequence.

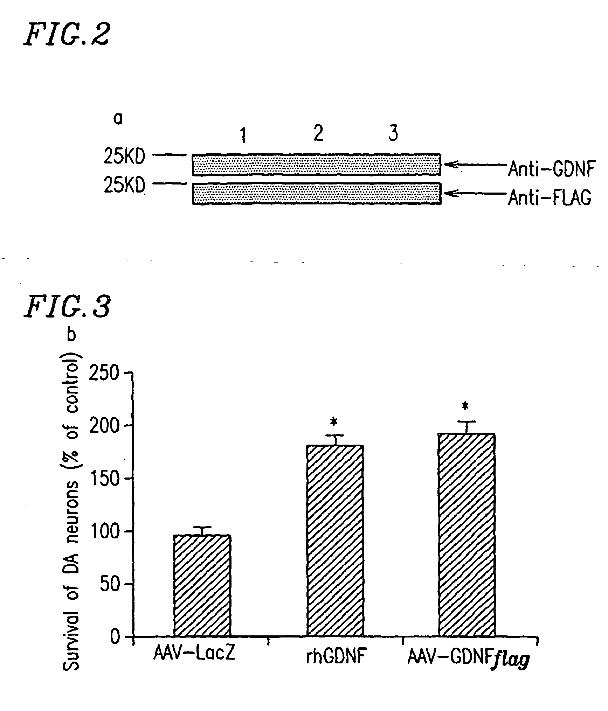

Figure 2 is a representation of a Western blot analysis of cell extracts from 293 cells after transduction with AAV-GDNF*flag*. 293 cells transduced with mock (lane 1) or AAV-LacZ (lane 2) were used as negative controls. Thirty-six hours after transduction with AAV-GDNF*flag* (lane 3), expression of GDNF*flag* fusion protein was detected by anti-GDNF and anti-FLAG antibody, respectively.
Figure 3 shows increased survival of DA neurons in cultures transduced with AAV-GDNF*flag*. Number of TH-IR (DA) neurons was counted in rat E14 mesencephalic cell cultures after 9 days transduced with AAV-GDNF*flag*, AAV-*LacZ* or mock. The rhGDNF (10 ng/ml) was added to culture as positive control. Error bars indicate s.e.m. (*P < 0.01).

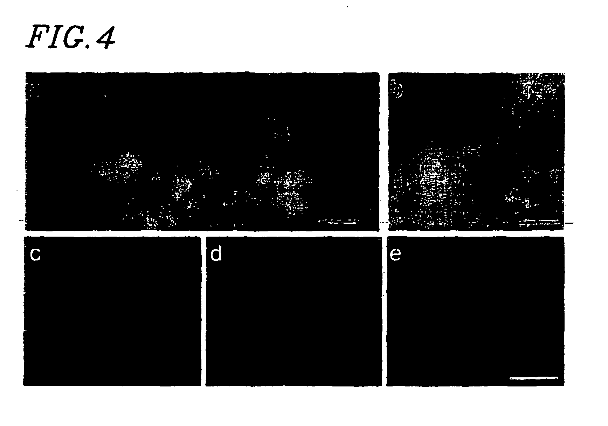

Figures 4a-4e depict expression of GDNF*flag* in striatum and retrograde transport to SN. Four weeks after AAV-GDNF*flag* was infected into lesioned striatum, rats were killed and coronal sections through striatum and SN were processed for immunohistochemistry. GDNF*flag* transgene expression was detected in striatum with anti-FLAG antibody (Figures 4a and 4b). Retrograde transport of GDNF*flag* in the ipsilateral SN was confirmed by double immunofluorescence staining with polyclonal anti-TH (4c) and monoclonal anti-FLAG (4d) antibodies. (4e) Overlay of Figures 4c and 4d shows co-localization of FLAG and TH-positive neurons in the SN. Double-positive neurons were detected in a broad area of pars compacta. This figure shows the area where most prominent co-localization of TH and FLAG was observed. (a, bar= 100 µm; b, bar=25 µm; c-e, bar=50 µm).

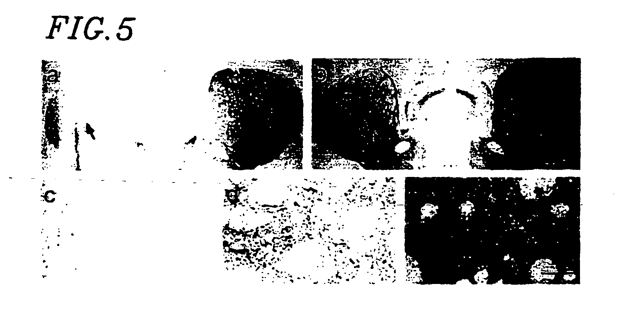

Figures 5a-5e show the effects of AAV-GDNF*flag* injection on TH-IR fibers in striatum: Densities of TH-IR fibers were markedly reduced on the lesioned side of AAV-*LacZ*-injected animals (Figures 5a and 5c). In contrast, AAV-GDNF*flag*-injected animals demonstrated increased densities of TH-IR fibers in treated striatum (Figures 5b and 5d). Figures 5c and 5d are high magnification views of areas indicated by arrows in the lesioned sides of Figures 5a and 5b, respectively. Figure 5e shows dense TH-IR fibers in the area indicated by the arrowhead on the intact side of Figure 5b. (Figures 5a-5b, bar=1 mm; Figures 5c-5e, bar= 50µm).

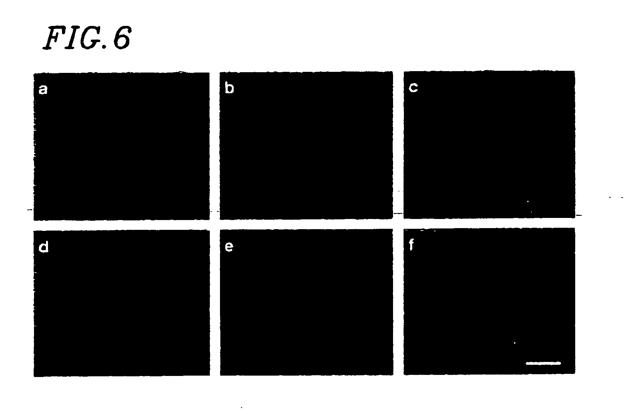

Figures 6a-6f show retrograde labeling with CTB, 4 weeks after creation of 6-OHDA lesion. Sections from SN were double-stained for CTB (Figures 6a and 6d) and TH (Figures 6b and 6e). CTB-positive cells, both in lesioned (Figures 6a-6c) and contralateral (Figures 6d-6f) side, were also TH-IR positive, representing DA neurons. On the lesioned side (Figures 6a-6c), TH-positive neurons were retrogradely labeled by CTB indicating some DA neurons maintained a connection to the striatal lesion at 4 weeks after lesion. Figure 6c is an overlay of Figures 6a and 6b. Figure 6f is an overlay of Figures 6d and 6e (bar=50 µm).

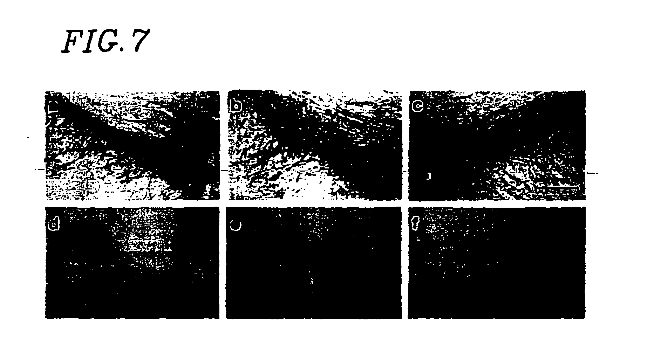

Figures 7a-7f show the effects of AAV-GDNF*flag* on the number of TH- and CTB-positive cells in SN. Coronal sections through midbrain revealed TH-IR (Figures 7a-7c) and CTB-positive (Figures 7d-7f) nigral neurons 20 weeks after AAV vector administration. CTB was bilaterally injected into striatum 3 days before the animals were killed. Examples of SN from the lesioned side in AAV-*LacZ*-injected animal (Figures 7a and 7d), and lesioned side (Figures 7b and 7e) and intact hemisphere (Figures 7c and 7f) in AAV-GDNF*flag*-injected animals, respectively. (Figures 7a-7c, bar=200 µm; Figures 7d-7f, bar =100 µm).

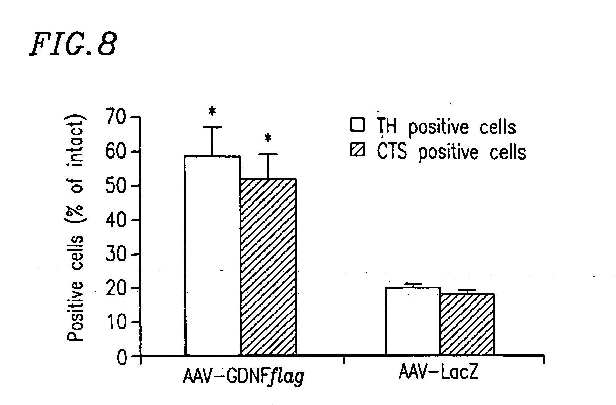

Figure 8 shows a significant increase in the percentage of TH-positive and CTB-positive neurons in the lesioned SN of the rats that received an AAV-GDNF*flag* injection as compared with AAV-*LacZ*-injected animals (n=8). Data were presented as the percentage of positive cells (lesioned versus unlesioned hemisphere, *P < 0.01).

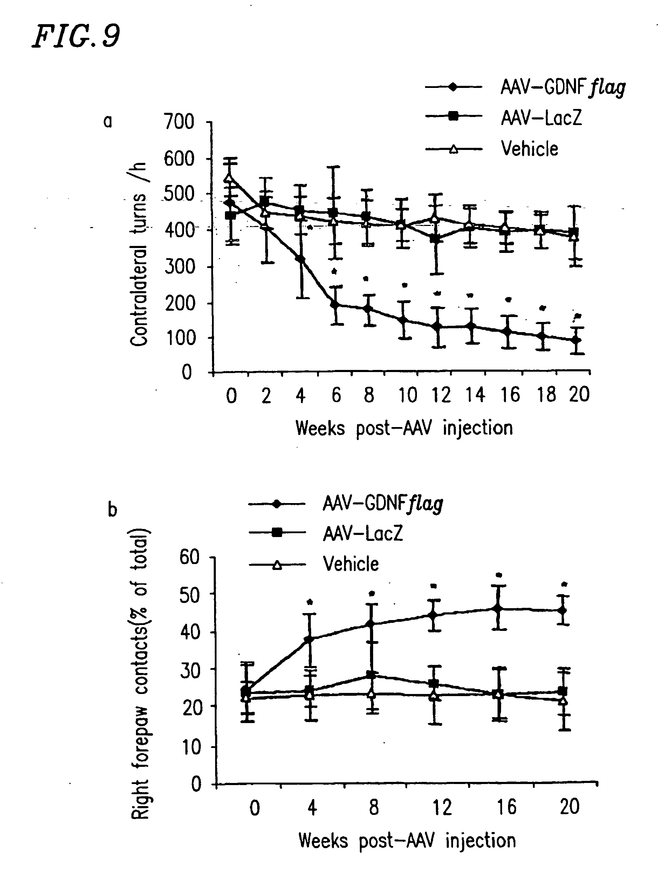

Figures 9a-9b show the effects of GDNF*flag* on behavioral recovery. Rats with intrastriatal injection of AAV-GDNF*flag* (n=20), AAV-*LacZ* (n=16) or vehicle (n=8) were examined for apomorphine-induced rotation and cylinder test. Significant decrease in apomorphine-induced rotations was observed in rats receiving AAV-GDNF*flag.* In contrast, rats in the AAV-*LacZ*-injection and vehicle injection groups demonstrated stable rotations. Rotations were expressed as turns per 60 min. (Figure 9a). Spontaneous forelimb use after 6-OHDA lesion was evaluated with the cylinder test. Before AAV treatment, rats demonstrated marked ipsilateral side bias, as indicated by reduced frequency of contralateral limb use (23-25% of total contacts). AAV-GDNF*flag*-treated rats improved gradually and reached near normal (47 % of total) at 20 weeks. In contrast, reduced frequency of contralateral limb use persisted in rats receiving AAV-*LacZ* or vehicle. Contralateral (right) forepaw contacts were presented as percentage of total (*P < 0.01). (Figure 9b).

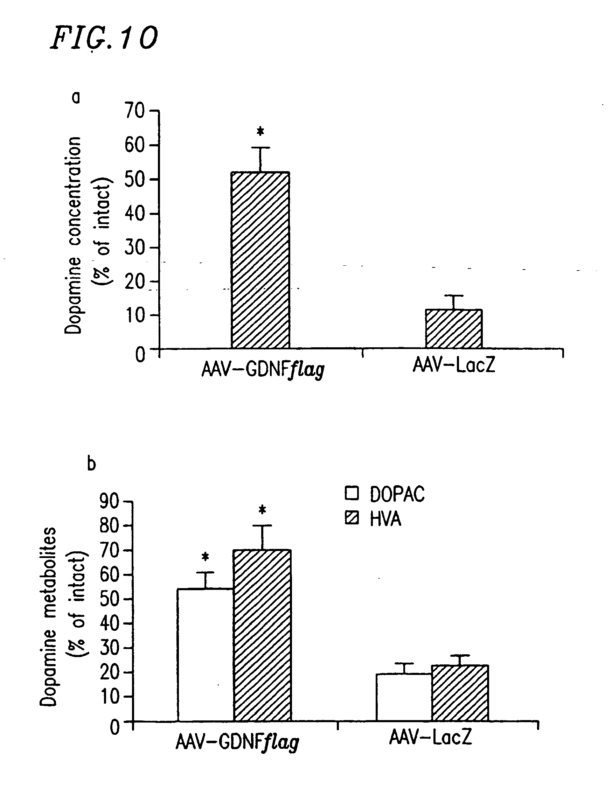

Figures 10a and 10b show Dopamine (Figure 10a) and DOPAC and HVA (dopamine metabolites) (Figure 10b) contents in 6-OHDA-lesioned striatum 20 weeks after AAV-GDNF*flag* (n=8) or AAV-*LacZ* (n=8) injection. Data represent percentage of intact striatum (P < 0.01).

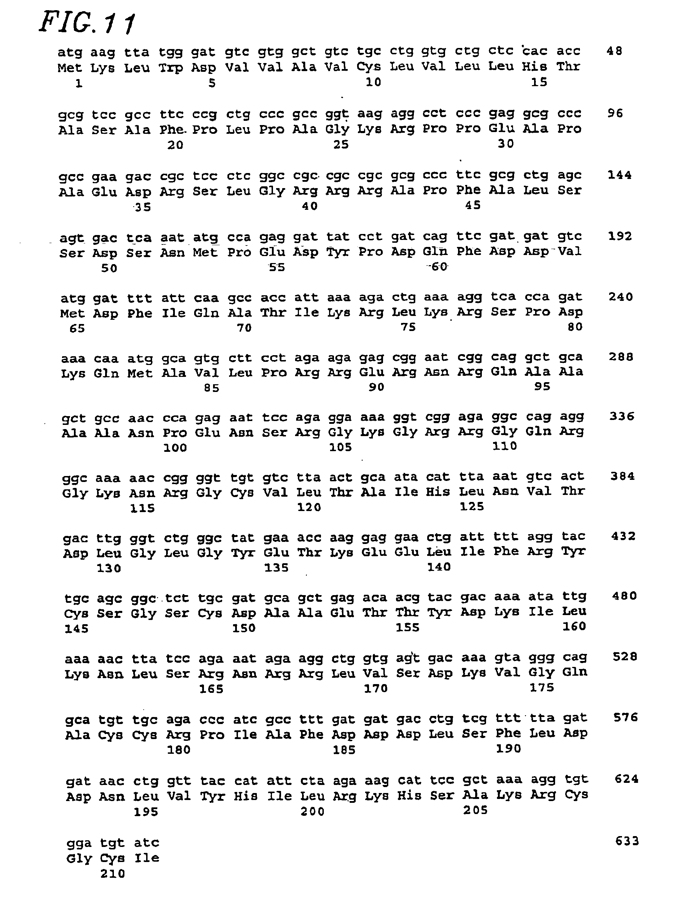

Figure 11 (SEQ ID NOS:1 and 2) shows the nucleotide sequence and amino acid sequence for a human pre-pro-GDNF. The mature GDNF molecule spans amino acid positions 78-211.

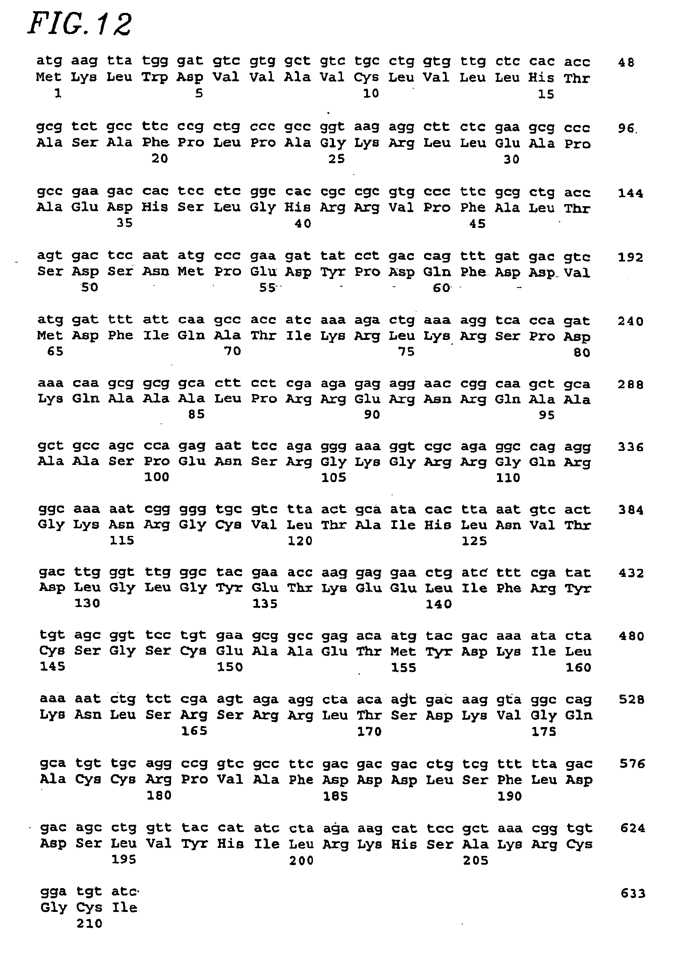

Figure 12 (SEQ ID NOS:3 and 4) shows the nucleotide sequence and amino acid sequence for a rat pre-pro-GDNF. The mature GDNF molecule spans amino acid positions 78-211.

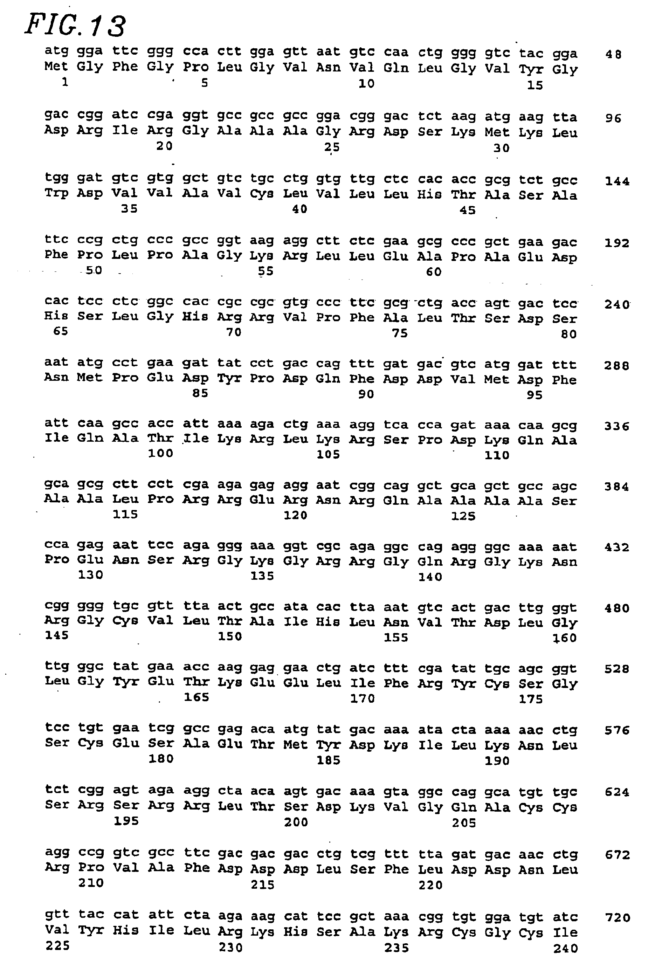

Figure 13 (SEQ ID NOS:6 and 7) shows the nucleotide sequence and amino acid sequence for a mouse pre-pro-GDNF. The mature GDNF molecule spans amino acid positions 107-240.

### Detailed Description of the Invention

The practice of the present invention will employ, unless otherwise indicated, conventional methods of virology, microbiology, molecular biology and recombinant DNA techniques within the skill of the art. Such techniques are explained fully in the literature. *See, e.g. ,* Sambrook et al. Molecular Cloning: A Laboratory Manual (Current Edition); DNA Cloning: A Practical Approach, Vol. I & II (D. Glover, ed.); Oligonucleotide Synthesis (N. Gait, ed., Current Edition); Nucleic Acid Hybridization (B. Hames & S. Higgins, eds., Current Edition); Transcription and Translation (B. Hames & S. Higgins, eds., Current Edition); CRC Handbook of Parvoviruses, vol. I & II (P. Tijssen, ed.); Fundamental Virology, 2nd Edition, vol. I & II (B.N. Fields and D.M. Knipe, eds.); Freshney Culture of Animal Cells, A Manual of Basic Technique (Wiley-Liss, Third Edition); and Ausubel et al. (1991) Current Protocols in Molecular Biology (Wiley Interscience, NY).

As used in this specification and the appended claims, the singular forms "a," "an" and "the" include plural references unless the content clearly dictates otherwise.

### A. Definitions

In describing the present invention, the following terms will be employed, and are intended to be defined as indicated below.

As used herein, the term "glial cell line-derived neurotrophic factor polypeptides" or "GDNF polypeptide" refers to a neurotrophic factor of any origin, which is substantially homologous and functionally equivalent to any of the various known GDNFs. Representative GDNFs for three mammalian species are shown in Figures 11, 12 and 13. The degree of homology between the rat (Figure 12, SEQ ID NO:4) and human (Figure 11, SEQ ID NO:2) protein is about 93% and all mammalian GDNFs have a similarly high degree of homology. Such GDNFs may exist as monomers, dimers or other multimers in their biologically active form. Thus, the term "GDNF polypeptide" as used herein encompasses active monomeric GDNFs, as well as active multimeric GDNFs, active glycosylated and non-glycosylated forms of GDNF and active truncated forms of the molecule.

By "functionally equivalent" as used herein, is meant a GDNF polypeptide that retains some or all of the neurotrophic properties, but not necessarily to the same degree, as a native GDNF molecule.

"Homology" refers to the percent similarity between two polynucleotide or two polypeptide moieties. Two polynucleotide, or two polypeptide sequences are "substantially homologous" to each other when the sequences exhibit at least about 50% , preferably at least about 75%, more preferably at least about 80%-85%, preferably at least about 90%, and most preferably at least about 95%-99% or more sequence similarity or sequence identity over a defined length of the molecules. As used herein, substantially homologous also refers to sequences showing complete identity to the specified polynucleotide or polypeptide sequence.

In general, "identity" refers to an exact nucleotide-to-nucleotide or amino acid-to-amino acid correspondence of two polynucleotides or polypeptide sequences, respectively. Percent identity can be determined by a direct comparison of the sequence information between two molecules by aligning the sequences, counting the exact number of matches between the two aligned sequences, dividing by the length of the shorter sequence, and multiplying the result by 100.

Readily available computer programs can be used to aid in the analysis of similarity and identity, such as ALIGN, Dayhoff, M.O. in Atlas of Protein Sequence and Structure M.O. Dayhoff ed., 5 Suppl. 3:353-358, National biomedical Research Foundation, Washington, DC, which adapts the local homology algorithm of Smith and Waterman Advances in Appl. Math. 2:482-489, 1981 for peptide analysis. Programs for determining nucleotide sequence similarity and identity are available in the Wisconsin Sequence Analysis Package, Version 8 (available from Genetics Computer Group, Madison, WI) for example, the BESTFIT, FASTA and GAP programs, which also rely on the Smith and Waterman algorithm. These programs are readily utilized with the default parameters recommended by the manufacturer and described in the Wisconsin Sequence Analysis Package referred to above. For example, percent similarity of a particular nucleotide sequence to a reference sequence can be determined using the homology algorithm of Smith and Waterman with a default scoring table and a gap penalty of six nucleotide positions.

Another method of establishing percent similarity in the context of the present invention is to use the MPSRCH package of programs copyrighted by the University of Edinburgh, developed by John F. Collins and Shane S. Sturrok, and distributed by IntelliGenetics, Inc. (Mountain View, CA). From this suite of packages the Smith-Waterman algorithm can be employed where default parameters are used for the scoring table (for example, gap open penalty of 12, gap extension penalty of one, and a gap of six). From the data generated the "Match" value reflects "sequence similarity." Other suitable programs for calculating the percent identity or similarity between sequences are generally known in the art, for example, another alignment program is BLAST, used with default parameters. For example, BLASTN and BLASTP can be used using the following default parameters: genetic code = standard; filter = none; strand = both; cutoff = 60; expect =10; Matrix = BLOSUM62; Descriptions = 50 sequences; sort by = HIGH SCORE; Databases = non-redundant, GenBank + EMBL + DDBJ + PDB + GenBank CDS translations + Swiss protein + Spupdate + PIR. Details of these programs can be found at the following internet address: http://www.ncbi.nlm.gov/cgi-bin/BLAST.

Alternatively, homology can be determined by hybridization of polynucleotides under conditions which form stable duplexes between homologous regions, followed by digestion with single-stranded-specific nuclease(s), and size determination of the digested fragments. DNA sequences that are substantially homologous can be identified in a Southern hybridization experiment under, for example, stringent conditions, as defined for that particular system. Defining appropriate hybridization conditions is within the skill of the art. See, e.g., Sambrook et al., *supra; DNA Cloning, supra; Nucleic Acid Hybridization, supra.*

By "GDNF variant" is meant a biologically active derivative of the reference GDNF molecule, or a fragment of such a derivative, that retains desired activity, such as neurotrophic activity in the assays described herein. In general, the term "variant" refers to compounds having a native polypeptide sequence and structure with one or more amino acid additions, substitutions (generally conservative in nature) and/or deletions, relative to the native molecule, so long as the modifications do not destroy neurotrophic activity. Preferably, the variant has at least the same neurotrophic activity as the native molecule. Methods for making polynucleotides encoding for GDNF variants are known in the art and are described further below.

For GDNF deletion variants, deletions generally range from about 1 to 30 residues, more usually from about 1 to 10 residues, and typically from about 1 to 5 contiguous residues, or any integer within the stated ranges. N-terminal, C-terminal and internal deletions are contemplated. Deletions are generally introduced into regions of low homology with other TGF-β super family members in order to preserve maximum biological activity. Deletions are typically selected so as to preserve the tertiary structure of the GDNF protein product in the affected domain, e.g., cysteine crosslinking. Non-limiting examples of deletion variants include truncated GDNF protein products lacking from 1-40 N-terminal amino acids of GDNF, or variants lacking the C-terminal residue of GDNF, or combinations thereof.

For GDNF addition variants, amino acid sequence additions typically include N-and/or C-terminal fusions ranging in length from one residue to polypeptides containing a hundred or more residues, as well as internal additions of single or multiple amino acid residues. Internal additions generally range from about 1-10 residues, more typically from about 1-5 residues, and usually from about 1-3 amino acid residues, or any integer within the stated ranges. Examples of N-terminal addition variants include the fusion of a heterologous N-terminal signal sequence to the N-terminus of GDNF as well as fusions of amino acid sequences derived from the sequence of other neurotrophic factors.

GDNF substitution variants have at least one amino acid residue of the GDNF amino acid sequence removed and a different residue inserted in its place. Such substitution variants include allelic variants, which are characterized by naturally occurring nucleotide sequence changes in the species population that may or may not result in an amino acid change. Particularly preferred substitutions are conservative in nature, i.e., those substitutions that take place within a family of amino acids that are related in their side chains. Specifically, amino acids are generally divided into four families: (1) acidic -- aspartate and glutamate; (2) basic -- lysine, arginine, histidine; (3) non-polar -- alanine, valine, leucine, isoleucine, proline, phenylalanine, methionine, tryptophan; and (4) uncharged polar -- glycine, asparagine, glutamine, cysteine, serine threonine, tyrosine. Phenylalanine, tryptophan, and tyrosine are sometimes classified as aromatic amino acids. For example, it is reasonably predictable that an isolated replacement of leucine with isoleucine or valine, an aspartate with a glutamate, a threonine with a serine, or a similar conservative replacement of an amino acid with a structurally related amino acid, will not have a major effect on the biological activity.

For example, the GDNF molecule may include up to about 5-10 conservative or non-conservative amino acid substitutions, or even up to about 15-25 conservative or non-conservative amino acid substitutions, or any integer between 5-25, so long as the desired function of the molecule remains intact. One of skill in the art may readily determine regions of the molecule of interest that can tolerate change using techniques well known in the art.

Specific mutations of the GDNF amino acid sequence may involve modifications to a glycosylation site (e.g., serine, threonine, or asparagine). The absence of glycosylation or only partial glycosylation results from amino acid substitution or deletion at any asparagine-linked glycosylation recognition site or at any site of the molecule that is modified by addition of an O-linked carbohydrate. An asparagine-linked glycosylation recognition site comprises a tripeptide sequence which is specifically recognized by appropriate cellular glycosylation enzymes. These tripeptide sequences are either Asn-Xaa-Thr or Asn-Xaa-Ser, where Xaa can be any amino acid other than Pro. A variety of amino acid substitutions or deletions at one or both of the first or third amino acid positions of a glycosylation recognition site (and/or amino acid deletion at the second position) result in non-glycosylation at the modified tripeptide sequence. Thus, the expression of appropriate altered nucleotide sequences produces variants which are not glycosylated at that site. Alternatively, the GDNF amino acid sequence may be modified to add glycosylation sites.

Methods for identifying GDNF amino acid residues or regions for mutagenesis are well known in the art. One such method is known as "alanine scanning mutagenesis."

See, e.g., Cunningham and Wells, Science (1989) 244:1081-1085. In this method, an amino acid residue or group of target residues are identified (e.g., charged residues such as Arg, Asp, His, Lys, and Glu) and replaced by a neutral or negatively charged amino acid (most preferably alanine or polyalanine) to affect the interaction of the amino acids with the surrounding aqueous environment in or outside the cell. Those domains demonstrating functional sensitivity to the substitutions are refined by introducing additional or alternate residues at the sites of substitution. Thus, the target site for introducing an amino acid sequence variation is determined, alanine scanning or random mutagenesis is conducted on the corresponding target codon or region of the DNA sequence, and the expressed GDNF variants are screened for the optimal combination of desired activity and degree of activity.

The sites of greatest interest for mutagenesis include sites where the amino acids found in GDNF proteins from various species are substantially different in terms of side-chain bulk, charge, and/or hydrophobicity. Other sites of interest are those in which particular residues of GDNF-like proteins, obtained from various species, are identical. Such positions are generally important for the biological activity of a protein. Initially, these sites are substituted in a relatively conservative manner. If such substitutions result in a change in biological activity, then more substantial changes (exemplary substitutions) are introduced, and/or other additions or deletions may be made, and the resulting products screened for activity.

Assays for GDNF activity are known in the art and include the ability to increase dopamine uptake in cultures of mesncephalic neurons and the ability to increase the survival of sympathetic ganglia neurons. See, e.g., U.S. Patent No. 6,362,319.

"Parkinson's disease-like symptoms" include muscle tremors, muscle weakness, rigidity, bradykinesia, alterations in posture and equilibrium, dimentia and like symptoms generally associated with Parkinson's disease or other neurodegenerative diseases.

By "vector" is meant any genetic element, such as a plasmid, phage, transposon, cosmid, chromosome, virus, virion, etc., which is capable of replication when associated with the proper control elements and which can transfer gene sequences between cells. Thus, the term includes cloning and expression vehicles, as well as viral vectors.

By an "AAV vector" is meant a vector derived from an adeno-associated virus serotype, including without limitation, AAV-1, AAV-2, AAV-3, AAV-4, AAV-5, AAV-6, AAV-7 and AAV-8. AAV vectors can have one or more of the AAV wild-type genes deleted in whole or part, preferably the *rep* and/or *cap* genes, but retain functional flanking ITR sequences. Functional ITR sequences are necessary for the rescue, replication and packaging of the AAV virion. Thus, an AAV vector is defined herein to include at least those sequences required in *cis* for replication and packaging (e.g., functional ITRs) of the virus. The ITRs need not be the wild-type nucleotide sequences, and may be altered, e.g., by the insertion, deletion or substitution of nucleotides, so long as the sequences provide for functional rescue, replication and packaging.

"AAV helper functions" refer to AAV-derived coding sequences which can be expressed to provide AAV gene products that, in turn, function in *trans* for productive AAV replication. Thus, AAV helper functions include both of the major AAV open reading frames (ORFs), *rep* and *cap.* The Rep expression products have been shown to possess many functions, including, among others: recognition, binding and nicking of the AAV origin of DNA replication; DNA helicase activity; and modulation of transcription from AAV (or other heterologous) promoters. The Cap expression products supply necessary packaging functions. AAV helper functions are used herein to complement AAV functions in *trans* that are missing from AAV vectors.

The term "AAV helper construct" refers generally to a nucleic acid molecule that includes nucleotide sequences providing AAV functions deleted from an AAV vector which is to be used to produce a transducing vector for delivery of a nucleotide sequence of interest. AAV helper constructs are commonly used to provide transient expression of AAV *rep* and/or *cap* genes to complement missing AAV functions that are necessary for lytic AAV replication; however, helper constructs lack AAV ITRs and can neither replicate nor package themselves. AAV helper constructs can be in the form of a plasmid, phage, transposon, cosmid, virus, or virion. A number of AAV helper constructs have been described, such as the commonly used plasmids pAAV/Ad and pIM29+45 which encode both Rep and Cap expression products. See, e.g., Samulski et al. (1989) J. Virol. 63:3822-3828; and McCarty et al. (1991) J. Virol. 65:2936-2945. A number of other vectors have been described which encode Rep and/or Cap expression products. See, e.g., U.S. Patent Nos. 5,139,941 and 6,376,237.

The term "accessory functions" refers to non-AAV derived viral and/or cellular functions upon which AAV is dependent for its replication. Thus, the term captures proteins and RNAs that are required in AAV replication, including those moieties involved in activation of AAV gene transcription, stage specific AAV mRNA splicing, AAV DNA replication, synthesis of Cap expression products and AAV capsid assembly. Viral-based accessory functions can be derived from any of the known helper viruses such as adenovirus, herpesvirus (other than herpes simplex virus type-1) and vaccinia virus.

The term "accessory function vector" refers generally to a nucleic acid molecule that includes nucleotide sequences providing accessory functions. An accessory function vector can be transfected into a suitable host cell, wherein the vector is then capable of supporting AAV virion production in the host cell. Expressly excluded from the term are infectious viral particles as they exist in nature, such as adenovirus, herpesvirus or vaccinia virus particles. Thus, accessory function vectors can be in the form of a plasmid, phage, transposon or cosmid.

In particular, it has been demonstrated that the full-complement of adenovirus genes are not required for accessory helper functions. In particular, adenovirus mutants incapable of DNA replication and late gene synthesis have been shown to be permissive for AAV replication. Ito et al., (1970) J. Gen. Virol. 9:243; Ishibashi et al, (1971) virology 45:317. Similarly, mutants within the E2B and E3 regions have been shown to support AAV replication, indicating that the E2B and E3 regions are probably not involved in providing accessory functions. Carter et al., (1983) Virology 126:505. However, adenoviruses defective in the E1 region, or having a deleted E4 region, are unable to support AAV replication. Thus, E1A and E4 regions are likely required for AAV replication, either directly or indirectly. Laughlin et al., (1982) J. Virol. 41:868; Janik et al., (1981) Proc. Natl. Acad. Sci. USA 78:1925; Carter et al., (1983) Virology 126:505. Other characterized Ad mutants include: E1B (Laughlin et al. (1982), *supra;* Janik et al. (1981), *supra;* Ostrove et al., (1980) Virology 104:502); E2A (Handa et al., (1975) J. Gen. Virol. 29:239; Strauss et al., (1976) J. Virol. 17:140; Myers et al., (1980) J. Virol. 35:665; Jay et al. , (1981) Proc. Natl. Acad. Sci. USA 78:2927; Myers et al., (1981) J. Biol. Chem. 256:567); E2B (Carter, Adeno-Associated Virus Helper Functions, in I CRC Handbook of Parvoviruses (P. Tijssen ed., 1990)); E3 (Carter et al. (1983), *supra*)*;* and E4 (Carter et al.(1983), *supra;* Carter (1995)). Although studies of the accessory functions provided by adenoviruses having mutations in the E1B coding region have produced conflicting results, Samulski et al., (1988) J. Virol. 62:206-210, recently reported than E1B55k is required for AAV virion production, while E1B19k is not. In addition, International Publication WO 97/17458 and Matshushita et al., (1998) Gene Therapy 5:938-945, describe accessory function vectors encoding various Ad genes.

Particularly preferred accessory function vectors comprise an adenovirus VA RNA coding region, an adenovirus E4 ORF6 coding region, an adenovirus E2A 72 kD coding region, an adenovirus E1A coding region, and an adenovirus E1B region lacking an intact E1B55k coding region. Such vectors are described in International Publication No. WO 01/83797.

By "capable of supporting efficient rAAV virion production" is meant the ability of an accessory function vector or system to provide accessory functions that are sufficient to complement rAAV virion production in a particular host cell at a level substantially equivalent to or greater than that which could be obtained upon infection of the host cell with an adenovirus helper virus. Thus, the ability of an accessory function vector or system to support efficient rAAV virion production can be determined by comparing rAAV virion titers obtained using the accessory vector or system with titers obtained using infection with an infectious adenovirus. More particularly, an accessory function vector or system supports efficient rAAV virion production substantially equivalent to, or greater than, that obtained using an infectious adenovirus when the amount of virions obtained from an equivalent number of host cells is not more than about 200 fold less than the amount obtained using adenovirus infection, more preferably not more than about 100 fold less, and most preferably equal to, or greater than, the amount obtained using adenovirus infection.

By "recombinant virus" is meant a virus that has been genetically altered, e.g., by the addition or insertion of a heterologous nucleic acid construct into the particle.

By "AAV virion" is meant a complete virus particle, such as a wild-type (wt) AAV virus particle (comprising a linear, single-stranded AAV nucleic acid genome associated with an AAV capsid protein coat). In this regard, single-stranded AAV nucleic acid molecules of either complementary sense, e.g., "sense" or "antisense" strands, can be packaged into any one AAV virion and both strands are equally infectious.

A "recombinant AAV virion," or "rAAV virion" is defined herein as an infectious, replication-defective virus including an AAV protein shell, encapsidating a heterologous nucleotide sequence of interest which is flanked on both sides by AAV ITRs. A rAAV virion is produced in a suitable host cell which has had an AAV vector, AAV helper functions and accessory functions introduced therein. In this manner, the host cell is rendered capable of encoding AAV polypeptides that are required for packaging the AAV vector (containing a recombinant nucleotide sequence of interest) into infectious recombinant virion particles for subsequent gene delivery.

The term "transfection" is used to refer to the uptake of foreign DNA by a cell, and a cell has been "transfected" when exogenous DNA has been introduced inside the cell membrane. A number of transfection techniques are generally known in the art. *See, e.g.,* Graham et al. (1973) Virology, 52:456, Sambrook et al. (1989) Molecular Cloning, a laboratory manual, Cold Spring Harbor Laboratories, New York, Davis et al. (1986) Basic Methods in Molecular Biology, Elsevier, and Chu et al. (1981) Gene 13:197. Such techniques can be used to introduce one or more exogenous DNA moieties, such as a nucleotide integration vector and other nucleic acid molecules, into suitable host cells.

The term "host cell" denotes, for example, microorganisms, yeast cells, insect cells, and mammalian cells, that can be, or have been, used as recipients of an AAV helper construct, an AAV vector plasmid, an accessory function vector, or other transfer DNA. The term includes the progeny of the original cell which has been transfected. Thus, a "host cell" as used herein generally refers to a cell which has been transfected with an exogenous DNA sequence. It is understood that the progeny of a single parental cell may not necessarily be completely identical in morphology or in genomic or total DNA complement as the original parent, due to natural, accidental, or deliberate mutation.

As used herein, the term "cell line" refers to a population of cells capable of continuous or prolonged growth and division *in vitro.* Often, cell lines are clonal populations derived from a single progenitor cell. It is further known in the art that spontaneous or induced changes can occur in karyotype during storage or transfer of such clonal populations. Therefore, cells derived from the cell line referred to may not be precisely identical to the ancestral cells or cultures, and the cell line referred to includes such variants.

The term "heterologous" as it relates to nucleic acid sequences such as coding sequences and control sequences, denotes sequences that are not normally joined together, and/or are not normally associated with a particular cell. Thus, a "heterologous" region of a nucleic acid construct or a vector is a segment of nucleic acid within or attached to another nucleic acid molecule that is not found in association with the other molecule in nature. For example, a heterologous region of a nucleic acid construct could include a coding sequence flanked by sequences not found in association with the coding sequence in nature. Another example of a heterologous coding sequence is a construct where the coding sequence itself is not found in nature (e.g., synthetic sequences having codons different from the native gene). Similarly, a cell transformed with a construct which is not normally present in the cell would be considered heterologous for purposes of this invention. Allelic variation or naturally occurring mutational events do not give rise to heterologous DNA, as used herein.

A "coding sequence" or a sequence which "encodes" a particular protein, is a nucleic acid sequence which is transcribed (in the case of DNA) and translated (in the case of mRNA) into a polypeptide *in vitro* or *in vivo* when placed under the control of appropriate regulatory sequences. The boundaries of the coding sequence are determined by a start codon at the 5' (amino) terminus and a translation stop codon at the 3' (carboxy) terminus. A coding sequence can include, but is not limited to, cDNA from prokaryotic or eukaryotic mRNA, genomic DNA sequences from prokaryotic or eukaryotic DNA, and even synthetic DNA sequences. A transcription termination sequence will usually be located 3' to the coding sequence.

A "nucleic acid" sequence refers to a DNA or RNA sequence. The term captures sequences that include any of the known base analogues of DNA and RNA such as, but not limited to 4-acetylcytosine, 8-hydroxy-N6-methyladenosine, aziridinylcytosine, pseudoisocytosine, 5-(carboxyhydroxylmethyl) uracil, 5-fluorouracil, 5-bromouracil, 5-carboxymethylaminomethyl-2-thiouracil, 5-carboxymethylaminomethyluracil, dihydrouracil, inosine, N6-isopentenyladenine, 1-methyladenine, 1-methylpseudouracil, 1-methylguanine, 1-methylinosine, 2,2-dimethylguanine, 2-methyladenine, 2-methylguanine, 3-methylcytosine, 5-methylcytosine, N6-methyladenine, 7-methylguanine, 5-methylaminomethyluracil, 5-methoxyaminomethyl-2-thiouracil, beta-D-mannosylqueosine, 5'-methoxycarbonylmethyluracil, 5-methoxyuracil, 2-methylthio-N6-isopentenyladenine, uracil-5-oxyacetic acid methylester, uracil-5-oxyacetic acid, oxybutoxosine, pseudouracil, queosine, 2-thiocytosine, 5-methyl-2-thiouracil, 2-thiouracil, 4-thiouracil, 5-methyluracil, uracil-5-oxyacetic acid methylester, uracil-5-oxyacetic acid, pseudouracil, queosine, 2-thiocytosine, and 2,6-diaminopurine.

The term DNA "control sequences" refers collectively to promoter sequences, polyadenylation signals, transcription termination sequences, upstream regulatory domains, origins of replication, internal ribosome entry sites ("IRES"), enhancers, and the like, which collectively provide for the replication, transcription and translation of a coding sequence in a recipient cell. Not all of these control sequences need always be present so long as the selected coding sequence is capable of being replicated, transcribed and translated in an appropriate host cell.

The term "promoter" is used herein in its ordinary sense to refer to a nucleotide region comprising a DNA regulatory sequence, wherein the regulatory sequence is derived from a gene which is capable of binding RNA polymerase and initiating transcription of a downstream (3'-direction) coding sequence. Transcription promoters can include "inducible promoters" (where expression of a polynucleotide sequence operably linked to the promoter is induced by an analyte, cofactor, regulatory protein, etc.), "repressible promoters" (where expression of a polynucleotide sequence operably linked to the promoter is induced by an analyte, cofactor, regulatory protein, etc.), and "constitutive promoters".

"Operably linked" refers to an arrangement of elements wherein the components so described are configured so as to perform their usual function. Thus, control sequences operably linked to a coding sequence are capable of effecting the expression of the coding sequence. The control sequences need not be contiguous with the coding sequence, so long as they function to direct the expression thereof. Thus, for example, intervening untranslated yet transcribed sequences can be present between a promoter sequence and the coding sequence and the promoter sequence can still be considered "operably linked" to the coding sequence.

By "isolated" when referring to a nucleotide sequence, is meant that the indicated molecule is present in the substantial absence of other biological macromolecules of the same type. Thus, an "isolated nucleic acid molecule which encodes a particular polypeptide" refers to a nucleic acid molecule which is substantially free of other nucleic acid molecules that do not encode the subject polypeptide; however, the molecule may include some additional bases or moieties which do not deleteriously affect the basic characteristics of the composition.

For the purpose of describing the relative position of nucleotide sequences in a particular nucleic acid molecule throughout the instant application, such as when a particular nucleotide sequence is described as being situated "upstream," "downstream," "3'," or "5"' relative to another sequence, it is to be understood that it is the position of the sequences in the "sense" or "coding" strand of a DNA molecule that is being referred to as is conventional in the art.

A "functional homologue," or a "functional equivalent" of a given AAV polypeptide includes molecules derived from the native polypeptide sequence, as well as recombinantly produced or chemically synthesized polypeptides which function in a manner similar to the reference AAV molecule to achieve a desired result. Thus, a functional homologue of AAV Rep68 or Rep78 encompasses derivatives and analogues of those polypeptides--including any single or multiple amino acid additions, substitutions and/or deletions occurring internally or at the amino or carboxy termini thereof-so long as integration activity remains.

A "functional homologue," or a "functional equivalent" of a given adenoviral nucleotide region includes similar regions derived from a heterologous adenovirus serotype, nucleotide regions derived from another virus or from a cellular source, as well as recombinantly produced or chemically synthesized polynucleotides which function in a manner similar to the reference nucleotide region to achieve a desired result. Thus, a functional homologue of an adenoviral VA RNA gene region or an adenoviral E2a gene region encompasses derivatives and analogues of such gene regions-including any single or multiple nucleotide base additions, substitutions and/or deletions occurring within the regions, so long as the homologue retains the ability to provide its inherent accessory function to support AAV virion production at levels detectable above background.

"Convection-enhanced delivery" refers to any non-manual delivery of agents. In the context of the present invention, examples of convection-enhanced delivery (CED) of AAV can be achieved by infusion pumps or by osmotic pumps. A more detailed description of CED is found below.

The term "central nervous system" or "CNS" includes all cells and tissue of the brain and spinal cord of a vertebrate. Thus, the term includes, but is not limited to, neuronal cells, glial cells, astrocytes, cereobrospinal fluid (CSF), interstitial spaces, bone, cartilage and the like. Regions of the CNS have been associated with various behaviors and/or functions. For example, the basal ganglia of the brain has been associated with motor functions, particularly voluntary movement. The basal ganglia is composed of six paired nuclei: the caudate nucleus, the putamen, the globus pallidus (or pallidum), the nucleus accumbens, the subthalamic nucleus and the substantia nigra. The caudate nucleus and putamen, although separated by the internal capsula, share cytoarchitechtonic, chemical and physiologic properties and are often referred to as the corpus striatum, or simply "the striatum." The substantia nigra, which degenerates in Parkinson's patients, provides major dopaminergic input into the basal ganglia.

The terms "subject", "individual" or "patient" are used interchangeably herein and refer to a vertebrate, preferably a mammal. Mammals include, but are not limited to, murines, simians, humans, farm animals, sport animals and pets.

An "effective amount" is an amount sufficient to effect beneficial or desired results. An effective amount can be administered in one or more administrations, applications or dosages.

### B. General Methods

The present invention is based on the surprising discovery that AAV vector-mediated GDNF gene delivery in a delayed manner reverses progressive degeneration of dopaminergic (DA) neurons, while preserving a functional nigrostriatal pathway. In particular, as detailed in the examples, an acceptable animal model for Parkinson's disease received injections of AAV vectors expressing GDNF tagged with FLAG peptide (AAV-GDNF*flag*) or β-galactosidase (AAV-*LacZ*) into the lesioned striatum. Immunostaining for FLAG demonstrated retrograde transport of GDNF*flag* to the substantia nigra (SN). The density of tyrosine hydroxylase (TH)-positive DA fibers in the striatum and the number of TH-positive or cholera toxin subunit B (CTB, neuronal tracer)-labeled neurons in the SN were significantly greater in the AAV-GDNF*flag* group than in the AAV-*LacZ* group. Dopamine levels and those of its metabolites in the striatum were remarkably higher in the AAV-GDNF*flag* group compared with the control group. Consistent with anatomical and biochemical changes, significant behavioral recovery was observed following AAV-GDNF*flag* injection. These data unexpectedly indicate that a delayed delivery of a GDNF gene using AAV-based delivery systems is efficacious even after the onset of progressive degeneration.

Accordingly, the present invention provides composition for treating neurodegenerative diseases and other nerve disorders after nerve damage has already occurred. In preferred embodiments, the nerve damage is due to Parkinson's disease or damaged or improperly functioning dopaminergic nerve cells.

Thus, the present invention can be used to deliver polynucleotides encoding GDNF polypeptides to patients suffering from any of several neurodegenerative disorders including, without limitation" Parkinson's disease (PD), amyotrophic lateral sclerosis (ALS), epilepsy, Alzheimer's disease, and the like, all the above disorders characterized by the existence in said disorder of pre-existing neuronal damage comprising moderate to extensive nigrostriatal dopaminergie (DA) denervation. In addition, the present invention can be used to deliver GDNF to patients suffering from nerve damage due to temporary or permanent cessation of blood flow to parts of the nervous system, as in stroke, or due to exposure to neurotoxins, such as the cancer chemotherapeutic agents taxol, cisplatin or vincristine, and AIDS chemotherapeutic agents ddI or ddC, as well as nerve damage from chronic metabolic diseases, such as diabetes or renal dysfunction, all above disorders characterized by the existence in said disorder of pre-existing neuronal damage comprising moderate to extensive nigrostratal dopaminergie (DA) denervation. In addition, the present .

As explained above, GDNF is a protein that may be identified in or obtained from glial cells and that exhibits neurotrophic activity. More specifically, GDNF is a dopaminergic neurotrophic protein that is characterized in part by its ability to increase dopamine uptake on the embryonic precursors of the substantia nigra dopaminergic neurons, and further by its ability to promote the survival of parasympathetic and sympathetic nerve cells.

GDNF is an approximately 39 kD glycosylated protein that exists as a homodimer in its native form. GDNF is initially translated as a pre-pro-GDNF polypeptide and proteolytic processing of the signal sequence and the "pro" portion of the molecule result in production of a mature form of GDNF. In humans and rodents, a single gene gives rise to alternatively spliced forms. See, e.g., U.S. Patent No. 6,362,319. Both forms contain a consensus signal peptide sequence and a consensus sequence for proteolytic processing. Proteolytic cleavage yields identical mature 134 amino acid residue forms. Thus, the GDNF polynucleotide for use in the present AAV vectors may encode either or both of these forms, may encode the entire pre-pro-molecule, the pre-molecule, the pro-molecule, the mature GDNF polypeptide, or biologically active variants of these forms, as defined above.

A number of GDNF polynucleotide and amino acid sequences are known. Three representative mammalian GDNF sequences are depicted in Figures 11, 12 and 13 herein. In particular, a human GDNF nucleotide and amino acid sequence is shown in Figure 11 (SEQ ID NOS:1 and 2). A rat GDNF nucleotide and amino acid sequence is shown in Figure 12 (SEQ ID NOS:3 and 4) and a mouse GDNF nucleotide and amino acid sequence is shown in Figure 13 (SEQ ID NOS:6 and 7). The degree of homology between the rat and human proteins is about 93% and all mammalian GDNFs have a similarly high degree of homology. Additional GDNF nucleotide and amino acid sequences are known in the art. See, e.g., U.S. Patent Nos. 6,221,376 and 6,363,319, and Lin et al., Science (1993) 260:1130-1132 for rat and human sequences, as well as NCBI accession numbers AY052832, AJ001896, AF053748, AF063586 and L19063 for human sequences; NCBI accession numbers AF184922, AF497634, X92495, NM019139 for rat sequences; NCBI accession number AF516767 for a giant panda sequence; NCBI accession numbers XM122804, NM010275, D8835151, D49921, U36449, U37459, U66195 for mouse sequences; NCBI accession number AF469665 for a Nipponia nippon sequence; NCBI accession number AF106678 for a Macaca mulatta sequence; and NCBI accession numbers NM131732 and AF329853 for zebrafish sequences. As explained above, any of these sequences, as well as variants thereof, such as sequences substantially homologous and functionally equivalent to these sequences, will find use in the present methods.

The efficacy of AAV-delivered GDNF polynucleotides can be tested in any of a number of animal models of the above diseases, known in the art. For example, the most extensively used animal models of Parkinson's disease replicate the neurodegeneration of dopaminergic neurons usually by administration of toxins. Unilateral injection of 6-hydroxydopamine (6-OHDA) into the substantia nigra of mice or rats results in neuronal loss in the ipsilateral striatum and substantia nigra pars compacta with little change in contralateral hemisphere. Similarly, methamphetamine-induced neurotoxicity results in neurodegeneration of dopaminergic and serotoninergic neurons and is considered by those of skill in the art to be closely aligned to the human condition. Efficacy of a therapeutic agent may be evaluated by behavioral outcome using the apomorphine-induced rotational behavior.

Another Parkinson's disease model is constructed using the neurotoxin N-methyl-4-phenyl-1,2,3,6,-tetrahydropyridine (MPTP). MPTP is administered to mammals, such as mice, rats, and monkeys. Administration of MPTP to monkeys results not only in loss of dopaminergic and serotoninergic neurons in substantia nigra pars compacta and striatum, but also in behavioral manifestations similar to those seen in human Parkinson's disease patients, such as akinesia and rigid posture. See, e.g., U.S. Patent No. 6,362,319.

In contrast to the above-described animal models of Parkinson's disease, a number of inbred strains of mice are available which demonstrate a gradual decline in dopaminergic cell numbers. For example, a D2 receptor-deficient mouse has been generated by homologous recombination whose behavioral characteristics resemble those of patients afflicted with Parkinson's disease. Fitzgerald et al. (1993) Brain Res. 608:247-258. A second example is the weaver mutant mouse which shows a gradual decline in mesenchephalic dopaminergic neuron numbers over time up to 40 % . Verina et al. (1997) Exp. Brain Res. 113:5-12; Adelbrecht et al. (1996) Mol. Brain Res. 43:291-300; Mitsumoto et al. (1994) Science 265:1107-1110.

The present examples used the Sauer and Oertel partial PD model (Sauer and Oertel, Neuroscience (1994) 59:401-415). In this model, intrastriatal injection of 6-OHDA induces progressive retrograde degeneration of DA neurons that starts between 1 to 2 weeks after lesioning and continues over 8 to 16 weeks. This ongoing depletion of DA neurons may be more similar to the disease process of PD and more appropriate as an animal model for therapeutic study than the complete model, which is constructed by destroying the medial forebrain bundle, thereby causing more rapid degeneration of DA neurons. In the experiments detailed below, rats had exhibited consistent behavioral deficits before vector injection. The appearance of apomorphine-induced rotations is generally assumed to represent ≥ 90% depletion of striatal dopamine content (Hudson et al., Brain Res. (1993) 626:167-174). However, studies on PD patients and animal models have indicated that there might be more surviving DA neurons than the levels of dopamine suggested (Javoy-Agid et al., Neuroscience (1990) 38:245-253; Fearnley and Lees, Brain (1991) 114:2283-2301; Schulzer et al., Brain (1994) 117:509-516). In the model used herein, the number of CTB-positive neurons on the lesioned side of SN was 28.9% of contralateral value at 4 weeks post-lesion. This is consistent with previous studies using Fluorogold (FG)-retrograde labeling that demonstrated 28.8% (35 days post-lesion) (Kozlowski et al., Exp. Neurol. (2000) 166:1-15) or 34% (4 weeks post-lesion) (Sauer and Oertel, Neuroscience (1994) 59:401-415) of FG-positive cells in the lesioned SN. In addition, most CTB-labeled neurons were TH-positive, suggesting that part of the nigrostriatal projection remained intact at the time of AAV vector injection. Without being bound by a particular theory, these remaining portions of intact nigrostriatal projections and DA neurons may serve as substrate for regeneration and functional recovery after GDNF gene delivery.

Animal models of other neurodegenerative diseases have been described and are useful for evaluating the therapeutic efficacy of AAV-delivered GDNF polynucleotides in the treatment of neurodegenerative disorders in addition to PD. For example, Martin et al. (1995) Brain Res. 683:172-178 describe an animal model of epilepsy, Matheson et al. (1997) NeuroReport 8:1739-1742 and Oppenheim et al. (1995) Nature 373:344-346 describe models of neurodegeneration that results from physical trauma, and Sagot et al. (1996) J. Neurosci. 16:2335-2341 describe a model of motor neuron degeneration in animals.

Recombinant AAV virions comprising GDNF coding sequences may be produced using a variety of art-recognized techniques described more fully below. Wild-type AAV and helper viruses may be used to provide the necessary replicative functions for producing rAAV virions (see, e.g., U.S. Patent No. 5,139,941). Alternatively, a plasmid, containing helper function genes, in combination with infection by one of the well-known helper viruses can be used as the source of replicative functions (see e.g., U.S. Patent No. 5,622,856 and U.S. Patent No. 5,139,941. Similarly, a plasmid, containing accessory function genes can be used in combination with infection by wild-type AAV, to provide the necessary replicative functions. These three approaches, when used in combination with a rAAV vector, are each sufficient to produce rAAV virions. Other approaches, well known in the art, can also be employed by the skilled artisan to produce rAAV virions.

In a preferred embodiment of the present invention, a triple transfection method (described in detail in U.S. Patent No. 6,001,650) is used to produce rAAV virions because this method does not require the use of an infectious helper virus, enabling rAAV virions to be produced without any detectable helper virus present. This is accomplished by use of three vectors for rAAV virion production: an AAV helper function vector, an accessory function vector, and a rAAV expression vector. One of skill in the art will appreciate, however, that the nucleic acid sequences encoded by these vectors can be provided on two or more vectors in various combinations.

As explained herein, the AAV helper function vector encodes the "AAV helper function" sequences (i.e., *rep* and *cap*), which function *in trans* for productive AAV replication and encapsidation. Preferably, the AAV helper function vector supports efficient AAV vector production without generating any detectable wt AAV virions (i.e., AAV virions containing functional *rep* and *cap* genes). An example of such a vector, pHLP19 is described in U.S. Patent No. 6,001,650. The *rep* and *cap* genes of the AAV helper function vector can be derived from any of the known AAV serotypes, as explained above. For example, the AAV helper function vector may have a *rep* gene derived from AAV-2 and a *cap* gene derived from AAV-6; one of skill in the art will recognize that other *rep* and *cap,* gene combinations are possible, the defining feature being the ability to support rAAV virion production.

The accessory function vector encodes nucleotide sequences for non-AAV derived viral and/or cellular functions upon which AAV is dependent for replication (i.e., "accessory functions"). The accessory functions include those functions required for AAV replication, including, without limitation, those moieties involved in activation of AAV gene transcription, stage specific AAV mRNA splicing, AAV DNA replication, synthesis of *cap* expression products, and AAV capsid assembly. Viral-based accessory functions can be derived from any of the well-known helper viruses such as adenovirus, herpesvirus (other than herpes simplex virus type-1), and vaccinia virus. In a preferred embodiment, the accessory function plasmid pLadeno5 is used (details regarding pLadeno5 are described in U.S. Patent No. 6,004,797). This plasmid provides a complete set of adenovirus accessory functions for AAV vector production, but lacks the components necessary to form replication-competent adenovirus.

In order to further an understanding of the invention, a more detailed discussion is provided below regarding recombinant AAV expression vectors, AAV helper and accessory functions, compositions comprising AAV virions, as well as delivery of virions.

### Recombinant AAV Expression Vectors

Recombinant AAV (rAAV) expression vectors are constructed using known techniques to at least provide as operatively linked components in the direction of transcription, control elements including a transcriptional initiation region, the GDNF polynucleotide of interest and a transcriptional termination region. The control elements are selected to be functional in a mammalian muscle cell. The resulting construct which contains the operatively linked components is bounded (5' and 3') with functional AAV ITR sequences.

The nucleotide sequences of AAV ITR regions are known. *See, e.g.,* Kotin, R.M. (1994) Human Gene Therapy 5:793-801; Berns, K.I. "Parvoviridae and their Replication" in Fundamental Virology, 2nd Edition, (B.N. Fields and D.M. Knipe, eds.) for the AAV-2 sequence. AAV ITRs used in the vectors of the invention need not have a wild-type nucleotide sequence, and may be altered, e.g., by the insertion, deletion or substitution of nucleotides. Additionally, AAV ITRs may be derived from any of several AAV serotypes, including without limitation, AAV-1, AAV-2, AAV-3, AAV-4, AAV-5, AAV-6, AAV-7 and AAV-8, etc. Furthermore, 5' and 3' ITRs which flank a selected nucleotide sequence in an AAV expression vector need not necessarily be identical or derived from the same AAV serotype or isolate, so long as they function as intended, i.e., to allow for excision and rescue of the sequence of interest from a host cell genome or vector, and to allow integration of the DNA molecule into the recipient cell genome when AAV Rep gene products are present in the cell.

Suitable GDNF polynucleotide molecules for use in AAV vectors will be less than about 5 kilobases (kb) in size. The selected polynucleotide sequence is operably linked to control elements that direct the transcription or expression thereof in the subject *in vivo.* Such control elements can comprise control sequences normally associated with the selected gene. Alternatively, heterologous control sequences can be employed. Useful heterologous control sequences generally include those derived from sequences encoding mammalian or viral genes. Examples include, but are not limited to, neuron-specific enolase promoter, a GFAP promoter, the SV40 early promoter, mouse mammary tumor virus LTR promoter, adenovirus major late promoter (Ad MLP); a herpes simplex virus (HSV) promoter, a cytomegalovirus (CMV) promoter such as the CMV immediate early promoter region (CMVIE), a rous sarcoma virus (RSV) promoter, synthetic promoters, hybrid promoters, and the like. In addition, sequences derived from nonviral genes, such as the murine metallothionein gene, will also find use herein. Such promoter sequences are commercially available from, e.g., Stratagene (San Diego, CA).

The AAV expression vector which harbors the GDNF polynucleotide molecule of interest bounded by AAV ITRs, can be constructed by directly inserting the selected sequence(s) into an AAV genome which has had the major AAV open reading frames ("ORFs") excised therefrom. Other portions of the AAV genome can also be deleted, so long as a sufficient portion of the ITRs remain to allow for replication and packaging functions. Such constructs can be designed using techniques well known in the art. See, e.g., U.S. Patent Nos. 5,173,414 and 5,139,941; International Publication Nos. WO 92/01070 (published 23 January 1992) and WO 93/03769 (published 4 March 1993); Lebkowski et al. (1988) Molec. Cell. Biol. 8:3988-3996; Vincent et al. (1990) Vaccines 90 (Cold Spring Harbor Laboratory Press); Carter (1992) Current Opinion in Biotechnology 3:533-539; Muzyczka (1992) Current Topics in Microbiol. and Immunol. 158:97-129; Kotin (1994) Human Gene Therapy 5:793-801; Shelling and Smith (1994) Gene Therapy 1:165-169; and Zhou et al. (1994) J. Exp. Med. 179:1867-1875.

Alternatively, AAV ITRs can be excised from the viral genome or from an AAV vector containing the same and fused 5' and 3' of a selected nucleic acid construct that is present in another vector using standard ligation techniques, such as those described in Sambrook et al., *supra.* For example, ligations can be accomplished in 20 mM Tris-Cl pH 7.5, 10 mM MgCl₂, 10 mM DTT, 33 µg/ml BSA, 10 mM-50 mM NaCl, and either 40 µM ATP, 0.01-0.02 (Weiss) units T4 DNA ligase at 0°C (for "sticky end" ligation) or 1 mM ATP, 0.3-0.6 (Weiss) units T4 DNA ligase at 14°C (for "blunt end" ligation). Intermolecular "sticky end" ligations are usually performed at 30-100 µg/ml total DNA concentrations (5-100 nM total end concentration). AAV vectors which contain ITRs have been described in, *e.g*., U.S. Patent no. 5,139,941. In particular, several AAV vectors are described therein which are available from the American Type Culture Collection ("ATCC") under Accession Numbers 53222, 53223, 53224, 53225 and 53226.

Additionally, chimeric genes can be produced synthetically to include AAV ITR sequences arranged 5' and 3' of one or more selected nucleic acid sequences. Preferred codons for expression of the chimeric gene sequence in mammalian muscle cells can be used. The complete chimeric sequence is assembled from overlapping oligonucleotides prepared by standard methods. *See, e.g.,* Edge (1981) Nature 292:756; Nambair et al. (1984) Science 223:1299; Jay et al. (1984) J. Biol. Chem. (1984) 259:6311.

For the purposes of the invention, suitable host cells for producing rAAV virions from the AAV expression vectors include microorganisms, yeast cells, insect cells, and mammalian cells, that can be, or have been, used as recipients of a heterologous DNA molecule and that are capable of growth in suspension culture. The term includes the progeny of the original cell which has been transfected. Thus, a "host cell" as used herein generally refers to a cell which has been transfected with an exogenous DNA sequence. Cells from the stable human cell line, 293 (readily available through, e.g., the American Type Culture Collection under Accession Number ATCC CRL1573) are preferred in the practice of the present invention. Particularly, the human cell line 293 is a human embryonic kidney cell line that has been transformed with adenovirus type-5 DNA fragments (Graham et al. (1977) J. Gen. Virol. 36:59), and expresses the adenoviral E1a and E1b genes (Aiello et al. (1979) Virology 94:460). The 293 cell line is readily transfected, and provides a particularly convenient platform in which to produce rAAV virions.

### AAV Helper Functions

Host cells containing the above-described AAV expression vectors must be rendered capable of providing AAV helper functions in order to replicate and encapsidate the nucleotide sequences flanked by the AAV ITRs to produce rAAV virions. AAV helper functions are generally AAV-derived coding sequences which can be expressed to provide AAV gene products that, in turn, function in *trans* for productive AAV replication. AAV helper functions are used herein to complement necessary AAV functions that are missing from the AAV expression vectors. Thus, AAV helper functions include one, or both of the major AAV ORFs, namely the *rep* and *cap* coding regions, or functional homologues thereof.

By "AAV *rep* coding region" is meant the art-recognized region of the AAV genome which encodes the replication proteins Rep 78, Rep 68, Rep 52 and Rep 40. These Rep expression products have been shown to possess many functions, including recognition, binding and nicking of the AAV origin of DNA replication, DNA helicase activity and modulation of transcription from AAV (or other heterologous) promoters. The Rep expression products are collectively required for replicating the AAV genome. For a description of the AAV *rep* coding region, *see, e.g.,* Muzyczka, N. (1992) Current Topics in Microbiol. and Immunol. 158:97-129; and Kotin, R.M. (1994) Human Gene Therapy 5:793-801. Suitable homologues of the AAV *rep* coding region include the human herpesvirus 6 (HHV-6) *rep* gene which is also known to mediate AAV-2 DNA replication (Thomson et al. (1994) Virology 204:304-311).

By "AAV *cap* coding region" is meant the art-recognized region of the AAV genome which encodes the capsid proteins VP1, VP2, and VP3, or functional homologues thereof. These Cap expression products supply the packaging functions which are collectively required for packaging the viral genome. For a description of the AAV *cap* coding region, *see, e.g.,* Muzyczka, N. and Kotin, R.M. (*supra*)*.*

AAV helper functions are introduced into the host cell by transfecting the host cell with an AAV helper construct either prior to, or concurrently with, the transfection of the AAV expression vector. AAV helper constructs are thus used to provide at least transient expression of AAV *rep* and/or *cap* genes to complement missing AAV functions that are necessary for productive AAV infection. AAV helper constructs lack AAV ITRs and can neither replicate nor package themselves.

These constructs can be in the form of a plasmid, phage, transposon, cosmid, virus, or virion. A number of AAV helper constructs have been described, such as the commonly used plasmids pAAV/Ad and pIM29+45 which encode both Rep and Cap expression products. *See, e.g.,* Samulski et al. (1989) J. Virol. 63:3822-3828; and McCarty et al. (1991) *J. Virol.* 65:2936-2945. A number of other vectors have been described which encode Rep and/or Cap expression products. *See, e.g.,* U.S. Patent No. 5,139,941.

Both AAV expression vectors and AAV helper constructs can be constructed to contain one or more optional selectable markers. Suitable markers include genes which confer antibiotic resistance or sensitivity to, impart color to, or change the antigenic characteristics of those cells which have been transfected with a nucleic acid construct containing the selectable marker when the cells are grown in an appropriate selective medium. Several selectable marker genes that are useful in the practice of the invention include the hygromycin B resistance gene (encoding Aminoglycoside phosphotranferase (APH)) that allows selection in mammalian cells by conferring resistance to G418 (available from Sigma, St. Louis, Mo.). Other suitable markers are known to those of skill in the art.

### AAV Accessory Functions

The host cell (or packaging cell) must also be rendered capable of providing nonAAV-derived functions, or "accessory functions," in order to produce rAAV virions. Accessory functions are nonAAV-derived viral and/or cellular functions upon which AAV is dependent for its replication. Thus, accessory functions include at least those nonAAV proteins and RNAs that are required in AAV replication, including those involved in activation of AAV gene transcription, stage specific AAV mRNA splicing, AAV DNA replication, synthesis of Cap expression products and AAV capsid assembly. Viral-based accessory functions can be derived from any of the known helper viruses.

In particular, accessory functions can be introduced into and then expressed in host cells using methods known to those of skill in the art. Typically, accessory functions are provided by infection of the host cells with an unrelated helper virus. A number of suitable helper viruses are known, including adenoviruses; herpesviruses such as herpes simplex virus types 1 and 2; and vaccinia viruses. Nonviral accessory functions will also find use herein, such as those provided by cell synchronization using any of various known agents. *See, e.g.,* Buller et al. (1981) J. Virol. 40:241; 247; McPherson et al. (1985) Virology 147:217-222; Schlehofer et al. (1986) Virology 152:110-117.

Alternatively, accessory functions can be provided using an accessory function vector as defined above. See, e.g., U.S. Patent No. 6,004,797 and International Publication No. WO 01/83797. Nucleic acid sequences providing the accessory functions can be obtained from natural sources, such as from the genome of an adenovirus particle, or constructed using recombinant or synthetic methods known in the art. As explained above, it has been demonstrated that the full-complement of adenovirus genes are not required for accessory helper functions. In particular, adenovirus mutants incapable of DNA replication and late gene synthesis have been shown to be permissive for AAV replication. Ito et al., (1970) J. Gen. Virol. 9:243; Ishibashi et al, (1971) Virology 45:317. Similarly, mutants within the E2B and E3 regions have been shown to support AAV replication, indicating that the E2B and E3 regions are probably not involved in providing accessory functions. Carter et al., (1983) Virology 126:505. However, adenoviruses defective in the E1 region, or having a deleted E4 region, are unable to support AAV replication. Thus, E1A and E4 regions are likely required for AAV replication, either directly or indirectly. Laughlin et al., (1982) J. Virol. 41:868; Janik et al., (1981) Proc. Natl. Acad. Sci. USA 78: 1925; Carter et al., (1983) Virology 126:505. Other characterized Ad mutants include: E1B (Laughlin et al. (1982), *supra;* Janik et al. (1981), *supra;* Ostrove et al., (1980) Virology 104:502); E2A (Handa et al., (1975) J. Gen. Virol. 29:239; Strauss et al., (1976) J. Virol. 17:140; Myers et al., (1980) J. Virol. 35:665; Jay et al., (1981) Proc. Natl. Acad. Sci. USA 78:2927; Myers et al., (1981) J. Biol. Chem. 256:567); E2B (Carter, Adeno-Associated Virus Helper Functions, in I CRC Handbook of Parvoviruses (P. Tijssen ed., 1990)); E3 (Carter et al. (1983), *supra*)*;* and E4 (Carter et al.(1983), *supra;* Carter (1995)). Although studies of the accessory functions provided by adenoviruses having mutations in the E1B coding region have produced conflicting results, Samulski et al., (1988) J. Virol. 62:206-210, recently reported that E1B55k is required for AAV virion production, while E1B19k is not. In addition, International Publication WO 97/17458 and Matshushita et al., (1998) Gene Therapy 5:938-945, describe accessory function vectors encoding various Ad genes.

Particularly preferred accessory function vectors comprise an adenovirus VA RNA coding region, an adenovirus E4 ORF6 coding region, an adenovirus E2A 72 kD coding region, an adenovirus E1A coding region, and an adenovirus E1B region lacking an intact E1B55k coding region. Such vectors are described in International Publication No. WO 01/83797.

As a consequence of the infection of the host cell with a helper virus, or transfection of the host cell with an accessory function vector, accessory functions are expressed which transactivate the AAV helper construct to produce AAV Rep and/or Cap proteins. The Rep expression products excise the recombinant DNA (including the DNA of interest) from the AAV expression vector. The Rep proteins also serve to duplicate the AAV genome. The expressed Cap proteins assemble into capsids, and the recombinant AAV genome is packaged into the capsids. Thus, productive AAV replication ensues, and the DNA is packaged into rAAV virions.

Following recombinant AAV replication, rAAV virions can be purified from the host cell using a variety of conventional purification methods, such as column chromatography, CsCl gradients, and the like. For example, a plurality of column purification steps can be used, such as purification over an anion exchange column, an affinity column and/or a cation exchange column. See, for example, International Publication No. WO 02/12455. Further, if infection is employed to express the accessory functions, residual helper virus can be inactivated, using known methods. For example, adenovirus can be inactivated by heating to temperatures of approximately 60°C for, e.g., 20 minutes or more. This treatment effectively inactivates only the helper virus since AAV is extremely heat stable while the helper adenovirus is heat labile.

The resulting rAAV virions containing the GDNF nucleotide sequence of interest can then be used for gene delivery using the techniques described below.

### Compositions

Compositions will comprise sufficient genetic material to produce a therapeutically effective amount of the GDNF of interest, i.e., an amount sufficient to reduce or ameliorate symptoms of the disease state in question or an amount sufficient to confer the desired benefit. The compositions will also contain a pharmaceutically acceptable excipient. Such excipients include any pharmaceutical agent that does not itself induce the production of antibodies harmful to the individual receiving the composition, and which may be administered without undue toxicity. Pharmaceutically acceptable excipients include, but are not limited to, sorbitol, any of the various TWEEN compounds, and liquids such as water, saline, glycerol and ethanol. Pharmaceutically acceptable salts can be included therein, for example, mineral acid salts such as hydrochlorides, hydrobromides, phosphates, sulfates, and the like; and the salts of organic acids such as acetates, propionates, malonates, benzoates, and the like. Additionally, auxiliary substances, such as wetting or emulsifying agents, pH buffering substances, and the like, may be present in such vehicles. A thorough discussion of pharmaceutically acceptable excipients is available in REMINGTON'S PHARMACEUTICAL SCIENCES (Mack Pub. Co., N.J. 1991).

One particularly useful formulation comprises recombinant AAV virions in combination with one or more dihydric or polyhydric alcohols, and, optionally, a detergent, such as a sorbitan ester. See, for example, International Publication No. WO 00/32233.

As is apparent to those skilled in the art in view of the teachings of this specification, an effective amount of viral vector which must be added can be empirically determined. Representative doses are detailed below. Administration can be effected in one dose, continuously or intermittently throughout the course of treatment. Methods of determining the most effective means and dosages of administration are well known to those of skill in the art and will vary with the viral vector, the composition of the therapy, the target cells, and the subject being treated. Single and multiple administrations can be carried out with the dose level and pattern being elected by the treating physician.

It should be understood that more than one transgene can be expressed by the delivered recombinant virion. Alternatively, separate vectors, each expressing one or more different transgenes, can also be delivered to the CNS as described herein. Furthermore, it is also intended that the viral vectors delivered by the methods of the present invention be combined with other suitable compositions and therapies. For instance, Parkinson's disease can be treated by coadministering a recombinant AAV virion expressing GDNF into the CNS (e.g., into the caudate nucleus or putamen of the striatum) and additional agents, such as AADC, dopamine precursors (e.g., L-dopa), inhibitors of dopamine synthesis (e.g., carbidopa), inhibitors of dopamine catabolism (e.g., MaOB inhibitors), dopamine agonists or antagonists can be administered prior or subsequent to or simultaneously with the recombinant virion encoding GDNF. For example, the gene encoding AADC can be coadministered to the CNS along with the gene encoding GDNF. Similarly, L-dopa and, optionally, carbidopa, may be administered systemically. In this way, the dopamine which is naturally depleted in PD patients, is restored, apparently by AADC which is able to convert L-dopa into dopamine. Where the transgene is under the control of an inducible promoter, certain systemically delivered compounds such as muristerone, ponasteron, tetracyline or aufin may be administered in order to regulate expression of the transgene.

### Delivery of AAV Virions

Recombinant AAV virions may be introduced into cells of the CNS using either *in vivo* or *in vitro* (also termed *ex vivo*) transduction techniques to treat preexisting neuronal damage. If transduced *in vitro,* the desired recipient cell will be removed from the subject, transduced with rAAV virions and reintroduced into the subject. Alternatively, syngeneic or xenogeneic cells can be used where those cells will not generate an inappropriate immune response in the subject. Additionally, neural progenitor cells can be transduced *in vitro* and then delivered to the CNS.

Suitable methods for the delivery and introduction of transduced cells into a subject have been described. For example; cells can be transduced *in vitro* by combining recombinant AAV virions with cells to be transduced in appropriate media, and those cells harboring the DNA of interest can be screened using conventional techniques such as Southern blots and/or PCR, or by using selectable markers. Transduced cells can then be formulated into pharmaceutical compositions, as described above, and the composition introduced into the subject by various techniques as described below, in one or more doses.

For *in vivo* delivery, the rAAV virions will be formulated into pharmaceutical compositions and one or more dosages may be administered directly in the indicated manner. A therapeutically effective dose will include on the order of from about 10⁶ to 10¹⁵ of the rAAV virions, more preferably 10⁷ to 10¹², and even more preferably about 10⁸ to 10¹⁰ of the rAAV virions (or viral genomes, also termed "vg"), or any value within these ranges. Generally, from 0.01 to 1 ml of composition will be delivered, preferably from 0.01 to about .5 ml, and preferably about 0.05 to about 0.3 ml, such as 0.08, 0.09, 0.1, 0.2, etc. and any integer within these ranges, of composition will be delivered.

Recombinant AAV virions or cells transduced *in vitro* may be delivered directly to the CNS or brain by injection into, e.g., the ventricular region, as well as to the striatum (e.g., the caudate nucleus or putamen of the striatum), spinal cord and neuromuscular junction, or cerebellar lobule, with a needle, catheter or related device, using neurosurgical techniques known in the art, such as by stereotactic injection (see, e.g., Stein et al., J Virol 73:3424-3429, 1999; Davidson et al., PNAS 97:3428-3432, 2000; Davidson et al., Nat.Genet. 3:219-223, 1993; and Alisky and Davidson, Hum. Gene Ther. 11:2315-2329, 2000). Cerebellar injections are complicated by the fact that stereotaxic coordinates cannot be used to precisely target the site of an injection; there is animal to animal variation in the size of cerebellar lobules, as well as their absolute three-dimensional orientation. Thus, cholera toxin subunit b (CTb) may be used to determine the exact location of the injection and reveal the pool of transducable neurons at an injection site. Injections may fill the molecular layer, Purkinje cell layer, granule cell layer and white matter of the arbor vitae but do not extend to the deep cerebellar nuclei.

One mode of administration to the CNS uses a convection-enhanced delivery (CED) system. In this way, recombinant virions can be delivered to many cells over large areas of the brain. Moreover, the delivered vectors efficiently express transgenes in CNS cells (e.g., neurons or glial cells). Any convection-enhanced delivery device may be appropriate for delivery of viral vectors. In a preferred embodiments, the device is an osmotic pump or an infusion pump. Both osmotic and infusion pumps are commercially available from a variety of suppliers, for example Alzet Corporation, Hamilton Corporation, Alza, Inc., Palo Alto, California). Typically, a viral vector is delivered via CED devices as follows. A catheter, cannula or other injection device is inserted into CNS tissue in the chosen subject. One of skill in the art could readily determine which general area of the CNS is an appropriate target. For example, when delivering AAV-GDNF to treat PD, the striatum is a suitable area of the brain to target. Stereotactic maps and positioning devices are available, for example from ASI Instruments, Warren, MI. Positioning may also be conducted by using anatomical maps obtained by CT and/or MRI imaging of the subject's brain to help guide the injection device to the chosen target. Moreover, because the methods described herein can be practiced such that relatively large areas of the brain take up the viral vectors, fewer infusion cannula are needed. Since surgical complications are related to the number of penetrations, this mode of delivery serves to reduce the side effects seen with conventional delivery techniques. For a detailed description regarding CED delivery, see U.S. Patent No. 6,309,634.

In the case of PD, delivery is to subjects that demonstrate existing nigrostriatal dopaminergic (DA) denervation, said denervation being moderate to extensive. Generally, by the time a subject exhibits visible symptoms of PD, moderate to extensive denervation has occurred and at least about 60%-70% to more than 90% of existing neurons have been lost. By "moderate" denervation is meant, therefore, a loss of at least about 60%-70% of neurons while "extensive" denervation refers to a loss of at least about 90% of neurons.

One measure of neuron loss is by referring to levels of dopamine activity in the CNS. Thus, "moderate" denervation refers to a loss of at least about 60%-70% of dopamine content in one or both striatal hemispheres while "extensive" denervation is reflected by a loss of at least about 90% in one or both striatal hemispheres. To measure dopamine content, a labeled tracer is administered to the subject. The detection of the label is indicative of dopamine activity. Preferably, the labeled tracer is one that can be viewed *in vivo* in the brain of a whole animal, for example, by positron emission tomograph (PET) scanning or other CNS imaging techniques. Suitable labels for the selected tracer include any composition detectable by spectroscopic, photochemical, immunochemical, electrical, optical or chemical means. Useful labels in the present invention include radiolabels (e.g., ¹⁸F, ³H, ¹²⁵I, ^{3S}S, ³²P, etc), enzymes, colorimeteric labels, fluorescent dyes, and the like. In a preferred embodiment, the label ¹⁸F is used with DOPA to quantify dopamine activity. Thus, in this embodiment, the degree of neuron loss is measured using [¹⁸F]-fluro-DOPA as the tracer. Another measure of dopamine activity uses the labeled tracer 6-[¹⁸F]-fluoro-L-m-tyrosine (¹⁸F-FMT). FMT binds to cells that utilize dopamine. See, e.g., U.S. Patent No. 6,309,634 for methods of measuring dopamine content *in viv*o.

Regeneration of neurons and hence treatment of disease may also be monitored by measuring dopamine levels as described above. By treatment of disease, as used herein, is meant the reduction or elimination of symptoms of the disease of interest, as well as the regeneration of neurons. Thus, dopamine levels prior and subsequent to treatment can be compared as a measure of neuron regeneration. Alternatively, visual symptoms of disease can be used as a measure of treatment.

### Neural Tissue Analysis and Biochemical Analysis

Tissues can be harvested from treated subjects, and processed for evaluation of neuronal degeneration, regeneration and differentiation using routine procedures. In this invention it is useful to evaluate, for example, various cells of the striatum and substantia nigra (SN), such as examining coronal sections of the striatum and SN. Measurements performed over time can indicate increasing correction of cells distant to the vector administration site.

Levels of dopamine and its metabolites, HVA and DOPAC, can be evaluated using high-performance liquid chromatography (HPLC) as described previously (Shen, Y., Hum. Gene Ther. (2000) 11:1509-1519). CSF can also be collected and evaluated for protein levels or enzyme activity, particularly if the vector encodes a secretable form of GDNF.

Subjects can also be tested for rotational behavior periodically by intraperitoneal injection of apomorphine-HCl.

### C. Experimental

Below are examples of specific embodiments for carrying out the present invention. The examples are offered for illustrative purposes only, and are not intended to limit the scope of the present invention in any way.

Efforts have been made to ensure accuracy with respect to numbers used (e.g., amounts, temperatures, etc.), but some experimental error and deviation should, of course, be allowed for.

### Materials and Methods

### Animals and surgical procedures

Adult male Wistar rats (weight 200 to 250 g) were maintained in a 12-h light/dark cycle in cages with *ad libitum* access to food and water. Experiments were conducted in accordance with the Jichi Medical School guidelines for animal care. The partial rat model of PD was prepared as described by Sauer and Oertel, Neuroscience (1994) 59:401-415. Briefly, rats received stereotactic injections of 20 µg 6-hydroxydopamine (6-OHDA; calculated as free base; Sigma, St. Louis MO) dissolved in 4 µl of ascorbate-saline (0.05%) in the left striatum at the following coordinates: anterior-posterior (AP) +1.0 mm, medial-lateral (ML) 3.0 mm, dorso-ventral (DV) -4.5 mm. The stereotactic coordinates were calculated relative to the bregma and dural surface. After 4 weeks, animals were tested for apomorphine-induced rotation (0.1 mg/kg administered intraperitoneally) and only those animals exhibiting seven or more contralateral rotations/min in a 60-min period were included in further study. To assess the extent of nigrostriatal degeneration, neural tracer CTB (1% in distilled H₂O, 1 µl; List Biological Laboratories, Campbell, CA, USA) was injected bilaterally into the striatum (n=5, AP=+1 mm, ML=3.0 mm, DV=-4.5 mm) 3 days before death. This was performed to retrogradely label the subpopulation of DA neurons in the SN maintaining functional projections to the striatum at 4 weeks after 6-OHDA lesion. PD animal models were randomly assigned to receive AAV-GDNF*flag* (n=32), AAV-*LacZ* (n=22), or vehicle (0.1 M PBS; n=8) injection in the left striatum at three different sites (2 µl per site, 10¹³ vector genome copies/ml) using the following coordinates: (AP=+1.5 mm, +1.0 mm and +0.5 mm; ML=2.6 mm, 3.0 mm and 3.2 mm; DV=4.5 mm). The injection rate was set at 1 µl/min and the needle was left in place for an additional 7 min before being slowly retracted. To evaluate retrograde transport of GDNF*flag* protein, some rats (at each time point: AAV-GDNF*flag*-injected group, n=4; AAV-*LacZ*-injected group, n=2) were killed for histology at 2, 4 and 8 weeks after AAV injection, respectively. Twenty weeks after vector injection, rats were randomly grouped and killed for biochemical or immunohistochemical analysis. In eight rats, CTB was injected bilaterally into the striatum 3 days before death.

### Behavioral testing

After AAV vector injection, rats were tested for rotational behavior every 2 weeks by intraperitoneal injection of apomorphine-HCl (0.1 mg/kg). The total number of complete body turns was counted during an observation period of ≥ 60 min, as described previously (Shen, Y., Hum. Gene Ther. (2000) 11:1509-1519). Spontaneous limb use was scored every 4 weeks using the cylinder test method (Kirik et al., J. Neurosci. (2000) 20:4686-4700). Rats were placed in a clear glass cylinder large enough for free movement. After they had performed 10 rears during which they placed at least one paw on the cylinder wall, the number of both forepaw contacts to the wall of the cylinder was counted at least 20 times. The data for contralateral forepaw contacts were given as a percentage of total.

### Biochemical analysis

The rats were killed by decapitation under sodium pentobarbital anesthesia and brains were immediately dissected and placed on dry ice. The striatum was punched out bilaterally using a sharp-edged, stainless steel tube. Wet tissue samples were weighed and stored at -80°C until subsequent analysis. Levels of dopamine and its metabolites, HVA and DOPAC, were estimated using high-performance liquid chromatography (HPLC) as described previously (Shen, Y., Hum. Gene Ther. (2000) 11:1509-1519).

### Immunohistochemistry

Rats were perfused with PBS under deep anesthesia followed by ice-cold 4% PFA. Brains were dissected, and then post-fixed for 4 h in 4% PFA. Coronal sections (30 µm) from the striatum and SN were treated for 30 min in 0.3% H2O2 in PBS and blocked with 3% fetal bovine serum (FBS) in PBS/0.1% Triton X-100 for 1 h. Sections were then incubated with primary antibodies for TH (1:1000, mouse anti-TH monoclonal antibody; Chemicon, Temecula, CA, USA), CTB (1:10 000, goat antiserum to CTB; List Biologic, Campbell, CA, USA) or FLAG (1:1000, anti-FLAG M2 antibody, Sigma, St. Louis, MO) at 4°C overnight. This was followed by incubation with biotinylated secondary antibodies (to species of primary antibodies) for 1 h at room temperature (1:400, Santa Cruz, Santa Cruz, CA). Sections were visualized with avidin-biotinylated peroxidase complex procedure (Vectastain ABC kits; Vector Laboratories, Burlingame, CA, USA) using 3,3-diaminobenzidine (DAB) as a chromogen.

For FLAG/TH double immunofluorescence staining, sections were sequentially incubated with blocking solution containing 5% normal goat serum in PBS for 1 h at room temperature, monoclonal mouse anti-FLAG antibody (1:250, Sigma) and polyclonal rabbit anti-TH antibody (1:500) overnight at 4°C, and rhodamine-conjugated goat anti-mouse IgG (1:200; Santa Cruz, Santa Cruz, CA) and FITC-conjugated goat anti-rabbit IgG (1:200; Santa Cruz, Santa Cruz, CA) for 2 h at room temperature. Sections were examined and viewed under confocal laser scanning microscopy (TCS NT; Leica, Heidelberg, Germany). For CTB/TH double-immunofluorescence staining, anti-CTB (1:5000, goat antiserum to CTB) and mouse anti-TH (1:500) antibodies were used.

For quantitative analyses of TH-positive neurons and CTB-labeled neurons, sections at 300-µm intervals throughout the SN bilaterally were counted. Optical density of TH-IR fibers was quantified on sections every 300 µm rostro-caudally throughout the striatum using NIH Image 1.59 software (Kozlowski et al., Exp. Neurol. (2000) 166:1-15).

### Statistical analysis

Results were presented as mean ±s.e.m. Data were statistically analyzed using repeated-measures analysis of variance (ANOVA) followed by Tukey honestly significance difference (HSD) test (StatView 5.0 software).

### Example 1

### Construction of Recombinant AAV Vectors

Recombinant AAV vectors expressing FLAG-tagged GDNF (AAV-GDNF*flag*, Figure 1a) or β-galactosidase (AAV-*LacZ*, Figure 1b) were constructed as follows. As 6-OHDA lesions or injections of apomorphine induce endogenous expression of GDNF (Ohta et al., Biochem. Biophys. Res. Commun. (2000) 272:18-22; Zhou et al., Neuroreport (2000) 11:3289-3293), it is necessary to distinguish transgene-derived GDNF from endogenous GDNF to evaluate the effect of gene delivery. Thus, an AAV vector that expresses FLAG-tagged GDNF was constructed.

AAV vector plasmid pAAV-GDNF*flag* was derived from a previously described pAAV-GDNF plasmid (Fan et al., Neurosci. Lett. (1998) 248:61-64). This plasmid contains the mouse GDNF cDNA (Matsushita et al., Gene (1997) 203:149-157 tagged by FLAG sequence (DYKDDDDK (SEQ ID NO:5) at the carboxyl terminus under the human cytomegalovirus (CMV) immediate-early promoter, with human growth hormone first intron and simian virus 40 (SV40) polyadenylation signal sequence between the inverted terminal repeats (ITR) of the AAV-2 genome.

AAV vector plasmid pAAV-*LacZ*, auxiliary plasmid pHLP19 and pladenol were described previously (Fan et al., Neurosci. Lett. (1998) 248:61-64). Pladeno5 is described in U.S. Patent No. 6,004,797. Subconfluent human 293 cells were-transiently transfected with vector plasmid and helper plasmid using the calcium phosphate coprecipitation method. Seventy-two hours after transfection, cells were harvested and lysed by three freeze and thaw cycles. AAV vectors (AAV-GDNF*flag* and AAV-*LacZ*) were purified using two sequential continuous CsCl gradients, as described previously (Matsushita, et al, Gene Therapy (1998) 5:938-945). The final particle titer of the AAV-GDNF*flag* was 1.6×10¹³ vector genome copies/ml and AAV-*LacZ* was 2.1×10¹³ vector genome copies/ml, as estimated by quantitative DNA dot-blot hybridization analysis.

### Example 2

### In vitro Expression of AAV-GDNFflag

To detect the *in vitro* expression of GDNF*flag* fusion protein, 293 cells were transduced with AAV-GDNF*flag* or AAV-*LacZ* (5000 vector genome copies/cell). Thirty-six hours after transduction, cell lysates were separated by 15% polyacrylamide gel electrophoresis and transferred to polyvinylidene difluoride membranes (Millipore, Bedford, MA, USA). Residual protein-binding sites were blocked with 5% nonfat dried milk in TBS for 1 h at room temperature. Membranes were incubated overnight at 4°C using mouse anti-FLAG M2 antibody (1:1000, Sigma, St Louis, MO) or rabbit anti-GDNF antibody (1:2000, Santa Cruz, Santa Cruz, CA), followed by incubation with a secondary anti-mouse or anti-rabbit conjugated to horseradish peroxidase (1:2500, Amersham, Arlington Heights, IL) for 2 h at room temperature. Chemiluminescent signals were detected by the ECL systems (Amersham).

On Western blot analysis, GDNF*flag* fusion protein was detected in the lysate of AAV-GDNF*flag*-transduced 293 cells by both anti-GDNF and anti-FLAG antibodies. No signals were detected in the lysate of AAV-LacZ-transduced cells (Figure 2).

To assay the biological activity of AAV vector-derived GDNF*flag* fusion protein, primary cultures of rat fetal DA neurons were prepared. Briefly, the ventral mesencephalon was dissected out from day 14 Wistar rat embryos, minced and incubated with 0.25% trypsin in PBS for 15 min. Isolated cells were plated in 35-mm dishes precoated with poly-L-lysine, at an initial cell density of 10⁵ cells/dish, and incubated at 37°C with Neurobasal-SFM Media (Gibco BRL) supplemented with B27 (Gibco BRL), 100 units/ml penicillin, 100 µg/ml streptomycin, 25 mM KCl, and 2 mM glutamine. In AAV vector-treated dishes, cultures were infected with AAV-GDNF*flag* or AAV-*LacZ* (5000 vector genome copies/cell). In the positive control dish or negative control dish, recombinant human GDNF (rhGDNF, Santa Cruz) or BSA was added to cultures at a final concentration of 10 ng/ml. Culture medium was refreshed by half fresh medium at 3-day intervals, with rhGDNF or BSA kept at the same concentration. After 9 days, cells were fixed with 4% paraformaldehyde (PFA) for 30 min and processed for immunocytochemical staining to examine the expression of GDNF*flag* and TH. The number of TH-IR cells present was counted as described (Sawada et al., J. Neurochem. (2000) 74:1175-1184).

Tyrosine hydroxylase (TH) immunostaining revealed that about twice as many TH-immunoreactive (TH-IR) cells survived in AAV-GDNF*flag* transduced cultures than in AAV-*LacZ* transduced cultures (Figure 3). These results confirmed that AAV-GDNF*flag* could drive production of the bioactive GDNF*flag* fusion protein *in vitro,* and that the addition of the FLAG peptide did not alter the neurotrophic effects of GDNF on DA neurons, allowing identification of exogenously expressed GDNF*flag* fusion protein by anti-FLAG antibody.

### Example 3

### Expression of GDNFflag in the Striatum and Retrograde Transport to SN

GDNF*flag* transgene expression was detected in the striatum with anti-FLAG antibody at 2, 4, 8 and 20 weeks after AAV-GDNF*flag* injection. FLAG immunoreactive (FLAG-IR) cells were detected around the injection sites throughout the striatum. These cells displayed the typical morphology of medium spiny neurons (Figures 4a and 4b). Furthermore, dual immunofluorescence staining with anti-TH and anti-FLAG antibodies detected double-positive cells in the pars compacta of ipsilateral SN at 4 and 8 weeks after intrastriatal AAV-GDNF*flag* injection (Figures 4c-4e). At 8 weeks after AAV vector injection, 13.1 ± 2.4% (n=4) of TH-IR cells overlapped with FLAG-IR cells dispersed through the SN, indicating some transgenic GDNF*flag* obtained access to DA neurons. No FLAG-IR cells were observed in the striatum or SN on the contralateral side, nor in these regions in AAV-*LacZ* or vehicle-injected rats. In AAV-*LacZ*-injected rats, although X-gal-positive cells were detected in the striatum from 2-20 weeks after injection, no β-galactosidase signals could be detected in SN.

These results indicate that AAV-GDNF*flag* is capable of driving the expression of GDNF*flag* fusion protein in the striatum, and that GDNF*flag* fusion protein, not the AAV vector itself, could be retrogradely transported to the DA neuron bodies in SN from terminals in the striatum.

### Example 4

### Rescue of DA Innervation in the Striatum

In order to determine whether AAV-GDNF*flag* would rescue DA neuron innervation in the lesioned striatum, the extent of TH-IR fiber terminals was examined on slices throughout the striatum at 300-µm intervals at 20 weeks after injection. In AAV-*LacZ-*injected rats, the density of TH-IR fibers in the lesioned striatum was remarkably reduced to only 13.5 ±3.0% of the intact side (Figures 5a and 5c). In contrast, in rats injected with AAV-GDNF*flag*, the density of TH-IR fibers was significantly greater, reaching nearly 48.7 ±7.3% of the intact side (Figures 5b, 5d and 5e). Maintained TH-IR fibers were distributed around the injection sites, indicating that innervation of TH-IR fiber was rescued by the GDNFflag expressed in the striatum.

CTB back-labeling is more accurate in the assessment of nigrostriatal function than anti-TH immunostaining alone, because CTB labels only the SN neurons that maintain functional nigrostriatal projections, while anti-TH immunostaining merely demonstrates the presence of TH enzyme in neuron bodies. In the AAV-GDNF*flag* group, more CTB-labeled DA neurons existed than in the SN, suggesting that GDN*Fflag* expressed in the striatum could rescue DA neurons and promote functional projections to the striatum. In addition, successful rescue of the nigrostriatal system by intrastriatal AAV-GDNF*flag* injection was supported by high levels of dopamine and its metabolites in the striatum, indicating long-lasting activation of dopamine in the nigrostriatal system.

### Example 5

### Maintenance of Nigrostriatal Projections

Cholera toxin subunit B (CTB, neuronal tracer) (Leman et al., Brain Res. Brain Res. Protoc. (2000) 5:298-304) was injected bilaterally into the striatum to assess the extent of the remaining nigrostriatal pathway. The same coordinate of the 6-OHDA injection was utilized 3 days before death, to retrogradely label a subpopulation of DA neurons maintaining projections to the striatum. At the time point of AAV vector treatment, i.e., 4 weeks after 6-OHDA injection, the number of CTB-positive neurons in the lesioned SN was 28.9 ±3.7% of the intact contralateral side (n=5). Double immunofluorescence with anti-CTB and anti-TH antibodies revealed that most large CTB-positive neurons in SN were also TH-positive (Figure 6). The percentage of TH-IR neurons on the lesioned side was 34.9 ±2.6% (n=4) of the intact side. These results demonstrate that some portions of nigrostriatal connections remained at 4 weeks after lesion. At 20 weeks after the AAV vector injection, extensive loss of TH-IR cells (19.5 ±2.0% of intact side) and CTB-positive cells (17.9±1.6% of intact side) was observed in rats injected with AAV*-LacZ.* In contrast, injection of AAV-GDNF*flag* resulted in a significant increase in TH-IR neurons and CTB-positive cells, reaching 57.3 ±6.2% and 50.8 ±8.5%, respectively, of the contralateral side (Figures 7 and 8, P<0.01).

These results demonstrate that intrastriatal AAV-GDNF*flag* injection at 4-weeks post-lesion rescued the survival of DA neurons in the SN and promoted the maintenance of nigrostriatal projections.

### Example 6

### Behavioral Recovery After Intrastriatal Injection of AAV-GDNFflag

Four weeks after 6-OHDA administration, all rats in the three groups (AAV-GDNF*flag*, n=20; AAV-*LacZ,* n=16; and vehicle, n=8) demonstrated a similar degree of impairment both in the apomorphine-induced rotation tests and in the cylinder tests, suggesting equivalent levels of DA depletion.

A decrease in apomorphine-induced rotations was observed in rats receiving AAV-GDNF*flag*, beginning at 4 weeks after the vector injection (Figure 9a). This decrease proceeded gradually and persisted throughout the experiment (20 weeks). In contrast, rats in the AAV-*LacZ* or vehicle injection group demonstrated a fairly stable rotation rate during the same period.

The cylinder test was used to measure spontaneous forelimb use in lesioned rats (Figure 9b). Before AAV treatment, rats in each group showed a similar marked ipsilateral bias after a 6-OHDA lesion, as indicated by a reduced frequency of contralateral limb use (23-25% of total contacts) in this test. In rats receiving AAV-GDNF*flag*, significant improvement was observed from 4 weeks after injection, developing gradually and reaching near normal levels (47% of total contacts) at 20 weeks. In contrast, contralateral use persisted at the same level throughout the 20 weeks in rats receiving AAV-*LacZ* or vehicle. Statistical analysis of AAV-GDNF*flag* injected rats *versus* AAV-LacZ or vehicle-injected rats for both apomorphine-induced rotation and cylinder test revealed significant differences (P< 0.01).

### Example 7

### Preservation of Dopamine Content in the Lesioned Striatum

To test whether the behavioral recovery following AAV-GDNF*flag* injection correlated with nigrostriatal dopamine production, dopamine levels and those of its metabolites in the striatum, homovanillic acid (HVA) and 3,4-dihydroxyphenylacetic acid (DOPAC), were assayed at 20-weeks after injection. In AAV -GDNF*flag*-injected group (n=8), the dopamine level on the lesioned side of the striatum was 50.9 +7.7% of the contralateral intact side, while in the AAV-*LacZ* injected group (n=8) it remained at 11.5 ±4.8% of the contralateral side (Figure 10, P<0.01). In addition, levels of HVA and DOPAC were higher in the AAV-GDNF*flag*-injected group. No significant differences in levels of dopamine or its metabolites were observed on the intact side of striatum between the two groups.

The examples detailed above indicate that AAV vector-mediated delivery of a GDNF gene into the striatum of an acceptable animal model of PD protects SN neurons from further late degeneration, while maintaining their connection to the striatum.

In the present study, GDNF*flag* was detected in SN cells ipsilateral to the AAV-GDNFflag injection side from 4-8 weeks after injection. These SN cells were confirmed to be DA cells using double immunofluorescent staining with anti-FLAG and anti-TH antibodies. FLAG-IR cells were observed in a broad area of the pars compacta at 8 weeks after AAV vector treatment, although the percentage of FLAG-IR cells seemed low (13.1% of TH-IR cells). As GDNF exhibits extremely potent effects in the picomolar range on DA neurons, small amounts of GDNF*flag* protein transported transiently and not detected by immunohistochemistry at the time of death might protect nigral DA neurons against toxin-induced cell death. In contrast, rats injected with AAV*-LacZ* in the striatum demonstrated no β-galactosidase signal in the SN. In agreement with a previous study (Chamberlin et al., Brain Res. (1998) 793:169-175), AAV vector *per se* was not retrogradely transported in the central nervous system, but GDNF*flag* protein was retrogradely transported from the striatum to the DA neurons in the SN. Substantial expression of GDNF*flag* protein in the striatum was also detected at 20 weeks after injection. This persistence of GDNF*flag* production provided long-term rescue of the nigrostriatal system after a single AAV vector injection. The density of TH-IR fibers in the lesioned striatum was preserved after the AAV-GDNF*flag* injection.

Thus, AAV-mediated GDNF gene delivery in the striatum is efficacious as a neuroprotective gene therapy of PD and possibly, other CNS degenerative diseases.

### Industrial Applicability

Compositions for treating subjects with preexisting neuronal damage are disclosed. The compositions use adeno-associated virus (AAV)-based gene delivery systems for delivering glial cell line-derived neutrotrophic factor (GDNF) to subjects with neurodegenerative conditions such as Parkinson's disease.

## Claims

1. Use of recombinant adeno-associated virus (AAV) virions, wherein said virions comprise a polynucleotide encoding a glial cell line-derived neutrotrophic factor (GDNF) polypeptide operably linked to expression control elements that comprise a promoter, for the preparation of a medicament for the treatment in a mammalian subject of a central nervous system disorder **characterized by** the existence in said disorder of pre-existing neuronal damage comprising moderate to extensive nigrostriatal dopaminergic (DA) denervation.

2. The use of claim 1, wherein said central nervous system disorder is Parkinson's disease (PD).

3. The use of any claims 1 and 2, wherein the medicament is in a form for administration to the striatum of said subject.

## Patentansprüche

1. Verwendung von rekombinanten Virionen eines Adenoassoziierten Virus (AAV), wobei die Virionen ein Polynukleotid umfassen, das für ein GDNF (Gliazelllinien-abgeleiteter neurotropher Faktor)-Polypeptid kodiert, funktionell verknüpft mit Expressionskontrollelementen, die einen Promotor umfassen, zur Herstellung eines Medikaments zur Behandlung einer Störung des zentralen Nervensystems in einem Säugetier, **gekennzeichnet durch** das Vorliegen einer bereits bestehenden neuronalen Schädigung in der Störung, umfassend moderate bis extensive nigrostriatale dopaminerge (DA) Denervation.

2. Verwendung gemäß Anspruch 1, wobei die Störung des zentralen Nervensystems Parkinson-Krankheit (PD) ist.

3. Verwendung gemäß einem der Ansprüche 1 und 2, wobei das Medikament in einer Form zur Verabreichung an das Striatum des Säugetiers vorliegt.

## Revendications

1. Utilisation de virions de virus adéno-associés (VAA) recombinants, dans laquelle lesdits virions comprennent un polynucléotide codant pour un polypeptide du facteur neurotrophique dérivé de lignées cellulaires gliales (GDNF) lié de manière opérationnelle à des éléments de contrôle de l'expression comprenant un promoteur, dans la préparation d'un médicament pour le traitement d'un sujet mammifère atteint d'un trouble du système nerveux central **caractérisé par** l'existence dans ledit trouble de dommages neuronaux pré-existants comprenant une dénervation dopaminergique (DA) du faisceau strio-nigral modérée à étendue.

2. Utilisation selon la revendication 1, dans laquelle ledit trouble du système nerveux central est la maladie de Parkinson (PD).

3. Utilisation selon l'une quelconque des revendications 1 et 2, dans laquelle le médicament est sous une forme destinée à être administrée dans le striatum dudit sujet.


### SEQUENCE LISTING

<110> OZAWA, Keiya
   MURAMATSU, Shin-ichi
<120> COMPOSITIONS AND METHODS FOR TREATING
   NEURODEGENERATIVE DISEASES
<130> 0800-0028.40
<140> unassigned
   <141> 2002-08-28
<150> 60/315,838
   <151> 2001-08-29
<160> 7
<170> PatentIn Ver. 2.0
<210> 1
   <211> 633
   <212> DNA
   <213> Artificial Sequence
<220>
   <221> CDS
   <222> (1)..(633)
<220>
   <223> Description of Artificial Sequence: human GDNF
<400> 1
   atg aag tta tgg gat gtc gtg gct gtc tgc ctg gtg ctg ctc cac acc    48
   Met Lys Leu Trp Asp Val Val Ala Val Cys Leu Val Leu Leu His Thr
     1               5                  10                  15
   gcg tcc gcc ttc ccg ctg ccc gcc ggt aag agg cct ccc gag gcg ccc    96
   Ala Ser Ala Phe Pro Leu Pro Ala Gly Lys Arg Pro Pro Glu Ala Pro
                20                  25                  30
   gcc gaa gac cgc tcc ctc ggc cgc cgc cgc gcg ccc ttc gcg ctg agc    144
   Ala Glu Asp Arg Ser Leu Gly Arg Arg Arg Ala Pro Phe Ala Leu Ser
            35                  40                  45
   agt gac tca aat atg cca gag gat tat cct gat cag ttc gat gat gtc    192
   Ser Asp Ser Asn Met Pro Glu Asp Tyr Pro Asp Gln Phe Asp Asp Val
        50                  55                  60
   atg gat ttt att caa gcc acc att aaa aga ctg aaa agg tca cca gat    240
   Met Asp Phe Ile Gln Ala Thr Ile Lys Arg Leu Lys Arg Ser Pro Asp
    65                  70                  75                  80
   aaa caa atg gca gtg ctt cct aga aga gag cgg aat cgg cag gct gca    288
   Lys Gln Met Ala Val Leu Pro Arg Arg Glu Arg Asn Arg Gln Ala Ala
                    85                  90                  95
   gct gcc aac cca gag aat tcc aga gga aaa ggt cgg aga ggc cag agg    336
   Ala Ala Asn Pro Glu Asn Ser Arg Gly Lys Gly Arg Arg Gly Gln Arg
               100                 105                 110
   ggc aaa aac cgg ggt tgt gtc tta act gca ata cat tta aat gtc act      384
   Gly Lys Asn Arg Gly Cys Val Leu Thr Ala Ile His Leu Asn Val Thr
           115                 120                 125
   gac ttg ggt ctg ggc tat gaa acc aag gag gaa ctg att ttt agg tac      432
   Asp Leu Gly Leu Gly Tyr Glu Thr Lys Glu Glu Leu Ile Phe Arg Tyr
       130                 135                 140
   tgc agc ggc tct tgc gat gca gct gag aca acg tac gac aaa ata ttg      480
   Cys Ser Gly Ser Cys Asp Ala Ala Glu Thr Thr Tyr Asp Lys Ile Leu
   145                 150                 155                 160
   aaa aac tta tcc aga aat aga agg ctg gtg agt gac aaa gta ggg cag      528
   Lys Asn Leu Ser Arg Asn Arg Arg Leu Val Ser Asp Lys Val Gly Gln
                   165                 170                 175
   gca tgt tgc aga ccc atc gcc ttt gat gat gac ctg tcg ttt tta gat      576
   Ala Cys Cys Arg Pro Ile Ala Phe Asp Asp Asp Leu Ser Phe Leu Asp
               180                 185                 190
   gat aac ctg gtt tac cat att cta aga aag cat tcc gct aaa agg tgt      624
   Asp Asn Leu Val Tyr His Ile Leu Arg Lys His Ser Ala Lys Arg Cys
           195                 200                 205
   gga tgt atc                                                          633
   Gly Cys Ile
       210
<210> 2
   <211> 211
   <212> PRT
   <213> Artificial Sequence
<220>
   <223> Description of Artificial Sequence: human GDNF
<400> 2
   Met Lys Leu Trp Asp Val Val Ala Val Cys Leu Val Leu Leu His Thr
   1               5                   10                  15
   Ala Ser Ala Phe Pro Leu Pro Ala Gly Lys Arg Pro Pro Glu Ala Pro
               20                  25                  30
   Ala Glu Asp Arg Ser Leu Gly Arg Arg Arg Ala Pro Phe Ala Leu Ser
           35                  40                  45
   Ser Asp Ser Asn Met Pro Glu Asp Tyr Pro Asp Gln Phe Asp Asp Val
       50                  55                  60
   Met Asp Phe Ile Gln Ala Thr Ile Lys Arg Leu Lys Arg Ser Pro Asp
   65                  70                  75                  80
   Lys Gln Met Ala Val Leu Pro Arg Arg Glu Arg Asn Arg Gln Ala Ala
                   85                  90                  95
   Ala Ala Asn Pro Glu Asn Ser Arg Gly Lys Gly Arg Arg Gly Gln Arg
               100                 105                 110
   Gly Lys Asn Arg Gly Cys Val Leu Thr Ala Ile His Leu Asn Val Thr
           115                 120                 125
   Asp Leu Gly Leu Gly Tyr Glu Thr Lys Glu Glu Leu Ile Phe Arg Tyr
       130                 135                 140
   Cys Ser Gly Ser Cys Asp Ala Ala Glu Thr Thr Tyr Asp Lys Ile Leu
   145                 150                 155                 160
   Lys Asn Leu Ser Arg Asn Arg Arg Leu Val Ser Asp Lys Val Gly Gln
                   165                 170                 175
   Ala Cys Cys Arg Pro Ile Ala Phe Asp Asp Asp Leu Ser Phe Leu Asp
               180                 185                 190
   Asp Asn Leu Val Tyr His Ile Leu Arg Lys His Ser Ala Lys Arg Cys
           195                 200                 205
   Gly Cys Ile
       210
<210> 3
   <211> 633
   <212> DNA
   <213> Artificial Sequence
<220>
   <221> CDS
   <222> (1)..(633)
<220>
   <223> Description of Artificial Sequence: rat GDNF
<400> 3
   atg aag tta tgg gat gtc gtg gct gtc tgc ctg gtg ttg ctc cac acc    48
   Met Lys Leu Trp Asp Val Val Ala Val Cys Leu Val Leu Leu His Thr
    1               5                  10                  15
   gcg tct gcc ttc ccg ctg ccc gcc ggt aag agg ctt ctc gaa gcg ccc    96
   Ala Ser Ala Phe Pro Leu Pro Ala Gly Lys Arg Leu Leu Glu Ala Pro
               20                  25                  30
   gcc gaa gac cac tcc ctc ggc cac cgc cgc gtg ccc ttc gcg ctg acc   144
   Ala Glu Asp His Ser Leu Gly His Arg Arg Val Pro Phe Ala Leu Thr
           35                  40                  45
   agt gac tcc aat atg ccc gaa gat tat cct gac cag ttt gat gac gtc   192
   Ser Asp Ser Asn Met Pro Glu Asp Tyr Pro Asp Gln Phe Asp Asp Val
       50                  55                  60
   atg gat ttt att caa gcc acc atc aaa aga ctg aaa agg tca cca gat   240
   Met Asp Phe Ile Gln Ala Thr Ile Lys Arg Leu Lys Arg Ser Pro Asp
   65                  70                  75                  80
   aaa caa gcg gcg gca ctt cct cga aga gag agg aac cgg caa gct gca   288
   Lys Gln Ala Ala Ala Leu Pro Arg Arg Glu Arg Asn Arg Gln Ala Ala
                   85                  90                  95
   gct gcc agc cca gag aat tcc aga ggg aaa ggt cgc aga ggc cag agg   336
   Ala Ala Ser Pro Glu Asn Ser Arg Gly Lys Gly Arg Arg Gly Gln Arg
               100                 105                 110
   ggc aaa aat cgg ggg tgc gtc tta act gca ata cac tta aat gtc act   384
   Gly Lys Asn Arg Gly Cys Val Leu Thr Ala Ile His Leu Asn Val Thr
           115                 120                 125
   gac ttg ggt ttg ggc tac gaa acc aag gag gaa ctg atc ttt cga tat   432
   Asp Leu Gly Leu Gly Tyr Glu Thr Lys Glu Glu Leu Ile Phe Arg Tyr
       130                 135                 140
   tgt agc ggt tcc tgt gaa gcg gcc gag aca atg tac gac aaa ata cta   480
   Cys Ser Gly Ser Cys Glu Ala Ala Glu Thr Met Tyr Asp Lys Ile Leu
   145                 150                 155                 160
   aaa aat ctg tct cga agt aga agg cta aca agt gac aag gta ggc cag   528
   Lys Asn Leu Ser Arg Ser Arg Arg Leu Thr Ser Asp Lys Val Gly Gln
                   165                 170                 175
   gca tgt tgc agg ccg gtc gcc ttc gac gac gac ctg tcg ttt tta gac   576
   Ala Cys Cys Arg Pro Val Ala Phe Asp Asp Asp Leu Ser Phe Leu Asp
               180                 185                 190
   gac agc ctg gtt tac cat atc cta aga aag cat tcc gct aaa cgg tgt   624
   Asp Ser Leu Val Tyr His Ile Leu Arg Lys His Ser Ala Lys Arg Cys
           195                 200                 205
   gga tgt atc                                                       633
   Gly Cys Ile
       210
<210> 4
   <211> 211
   <212> PRT
   <213> Artificial Sequence
<220>
   <223> Description of Artificial Sequence: rat GDNF
<400> 4
   Met Lys Leu Trp Asp Val Val Ala Val Cys Leu Val Leu Leu His Thr
   1 5 10 15
   Ala Ser Ala Phe Pro Leu Pro Ala Gly Lys Arg Leu Leu Glu Ala Pro
   20 25 30
   Ala Glu Asp His Ser Leu Gly His Arg Arg Val Pro Phe Ala Leu Thr
   35 40 45
   Ser Asp Ser Asn Met Pro Glu Asp Tyr Pro Asp Gln Phe Asp Asp Val
   50 55 60
   Met Asp Phe Ile Gln Ala Thr Ile Lys Arg Leu Lys Arg Ser Pro Asp
   65 70 75 80
   Lys Gln Ala Ala Ala Leu Pro Arg Arg Glu Arg Asn Arg Gln Ala Ala
   85 90 95
   Ala Ala Ser Pro Glu Asn Ser Arg Gly Lys Gly Arg Arg Gly Gln Arg
   100 105 110
   Gly Lys Asn Arg Gly Cys Val Leu Thr Ala Ile His Leu Asn Val Thr
   115 120 125
   Asp Leu Gly Leu Gly Tyr Glu Thr Lys Glu Glu Leu Ile Phe Arg Tyr
   130 135 140
   Cys Ser Gly Ser Cys Glu Ala Ala Glu Thr Met Tyr Asp Lys Ile Leu
   145 150 155 160
   Lys Asn Leu Ser Arg Ser Arg Arg Leu Thr Ser Asp Lys Val Gly Gln
   165 170 175
   Ala Cys Cys Arg Pro Val Ala Phe Asp Asp Asp Leu Ser Phe Leu Asp
   180 185 190
   Asp Ser Leu Val Tyr His Ile Leu Arg Lys His Ser Ala Lys Arg Cys
   195 200 205
   Gly Cys Ile
   210
<210> 5
   <211> 8
   <212> PRT
   <213> Artificial Sequence
<220>
   <223> Description of Artificial Sequence: flag
<400> 5
   Asp Tyr Lys Asp Asp Asp Asp Lys
    1               5
<210> 6
   <211> 720
   <212> DNA
   <213> Artificial Sequence
<220>
   <221> CDS
   <222> (1) .. (720)
<220>
   <223> Description of Artificial Sequence: mouse GDNF
<400> 6
   atg gga ttc ggg cca ctt gga gtt aat gtc caa ctg ggg gtc tac gga   48
   Met Gly Phe Gly Pro Leu Gly Val Asn Val Gln Leu Gly Val Tyr Gly
    1               5                  10                  15
   gac cgg atc cga ggt gcc gcc gcc gga cgg gac tct aag atg aag tta   96
   Asp Arg Ile Arg Gly Ala Ala Ala Gly Arg Asp Ser Lys Met Lys Leu
               20                  25                  30
   tgg gat gtc gtg gct gtc tgc ctg gtg ttg ctc cac acc gcg tct gcc     144
   Trp Asp Val Val Ala Val Cys Leu Val Leu Leu His Thr Ala Ser Ala
           35                  40                  45
   ttc ccg ctg ccc gcc ggt aag agg ctt ctc gaa gcg ccc gct gaa gac     192
   Phe Pro Leu Pro Ala Gly Lys Arg Leu Leu Glu Ala Pro Ala Glu Asp
       50                  55                  60
   cac tcc ctc ggc cac cgc cgc gtg ccc ttc gcg ctg acc agt gac tcc     240
   His Ser Leu Gly His Arg Arg Val Pro Phe Ala Leu Thr Ser Asp Ser
   65                  70                  75                  80
   aat atg cct gaa gat tat cct gac cag ttt gat gac gtc atg gat ttt     288
   Asn Met Pro Glu Asp Tyr Pro Asp Gln Phe Asp Asp Val Met Asp Phe
                   85                  90                  95
   att caa gcc acc att aaa aga ctg aaa agg tca cca gat aaa caa gcg     336
   Ile Gln Ala Thr Ile Lys Arg Leu Lys Arg Ser Pro Asp Lys Gln Ala
               100                 105                 110
   gca gcg ctt cct cga aga gag agg aat cgg cag gct gca gct gcc agc     384
   Ala Ala Leu Pro Arg Arg Glu Arg Asn Arg Gln Ala Ala Ala Ala Ser
           115                 120                 125
   cca gag aat tcc aga ggg aaa ggt cgc aga ggc cag agg ggc aaa aat     432
   Pro Glu Asn Ser Arg Gly Lys Gly Arg Arg Gly Gln Arg Gly Lys Asn
       130                 135                 140
   cgg ggg tgc gtt tta act gcc ata cac tta aat gtc act gac ttg ggt     480
   Arg Gly Cys Val Leu Thr Ala Ile His Leu Asn Val Thr Asp Leu Gly
   145                 150                 155                 160
   ttg ggc tat gaa acc aag gag gaa ctg atc ttt cga tat tgc agc ggt     528
   Leu Gly Tyr Glu Thr Lys Glu Glu Leu Ile Phe Arg Tyr Cys Ser Gly
                   165                 170                 175
   tcc tgt gaa tcg gcc gag aca atg tat gac aaa ata cta aaa aac ctg     576
   Ser Cys Glu Ser Ala Glu Thr Met Tyr Asp Lys Ile Leu Lys Asn Leu
               180                 185                 190
   tct cgg agt aga agg cta aca agt gac aaa gta ggc cag gca tgt tgc     624
   Ser Arg Ser Arg Arg Leu Thr Ser Asp Lys Val Gly Gln Ala Cys Cys
           195                 200                 205
   agg ccg gtc gcc ttc gac gac gac ctg tcg ttt tta gat gac aac ctg     672
   Arg Pro Val Ala Phe Asp Asp Asp Leu Ser Phe Leu Asp Asp Asn Leu
       210                 215                 220
   gtt tac cat att cta aga aag cat tcc gct aaa cgg tgt gga tgt atc     720
   Val Tyr His Ile Leu Arg Lys His Ser Ala Lys Arg Cys Gly Cys Ile
   225                 230                 235                 240
<210> 7
   <211> 240
   <212> PRT
   <213> Artificial Sequence
<220>
   <223> Description of Artificial Sequence: mouse GDNF
<400> 7
   Met Gly Phe Gly Pro Leu Gly Val Asn Val Gln Leu Gly Val Tyr Gly
    1               5                  10                  15
   Asp Arg Ile Arg Gly Ala Ala Ala Gly Arg Asp Ser Lys Met Lys Leu
               20                  25                  30
   Trp Asp Val Val Ala Val Cys Leu Val Leu Leu His Thr Ala Ser Ala
           35                  40                  45
   Phe Pro Leu Pro Ala Gly Lys Arg Leu Leu Glu Ala Pro Ala Glu Asp
       50                  55                  60
   His Ser Leu Gly His Arg Arg Val Pro Phe Ala Leu Thr Ser Asp Ser
   65                  70                  75                  80
   Asn Met Pro Glu Asp Tyr Pro Asp Gln Phe Asp Asp Val Met Asp Phe
                   85                  90                  95
   Ile Gln Ala Thr Ile Lys Arg Leu Lys Arg Ser Pro Asp Lys Gln Ala
               100                 105                 110
   Ala Ala Leu Pro Arg Arg Glu Arg Asn Arg Gln Ala Ala Ala Ala Ser
           115                 120                 125
   Pro Glu Asn Ser Arg Gly Lys Gly Arg Arg Gly Gln Arg Gly Lys Asn
       130                 135                 140
   Arg Gly Cys Val Leu Thr Ala Ile His Leu Asn Val Thr Asp Leu Gly
   145                 150                 155                 160
   Leu Gly Tyr Glu Thr Lys Glu Glu Leu Ile Phe Arg Tyr Cys Ser Gly
                   165                 170                 175
   Ser Cys Glu Ser Ala Glu Thr Met Tyr Asp Lys Ile Leu Lys Asn Leu
               180                 185                 190
   Ser Arg Ser Arg Arg Leu Thr Ser Asp Lys Val Gly Gln Ala Cys Cys
           195                 200                 205
   Arg Pro Val Ala Phe Asp Asp Asp Leu Ser Phe Leu Asp Asp Asn Leu
       210                 215                 220
   Val Tyr His Ile Leu Arg Lys His Ser Ala Lys Arg Cys Gly Cys Ile
   225                 230                 235                 240